(19) Europäisches Patentamt
European Patent Office
Office européen des brevets

(11) **EP 3 074 568 B1**

(12) **EUROPÄISCHE PATENTSCHRIFT**

(45) Veröffentlichungstag und Bekanntmachung des Hinweises auf die Patenterteilung:
**28.03.2018 Patentblatt 2018/13**

(21) Anmeldenummer: **14781808.2**

(22) Anmeldetag: **18.09.2014**

(51) Int Cl.:
*D06M 11/00* (2006.01)     *D06M 13/184* (2006.01)
*D06M 13/192* (2006.01)     *D01D 10/02* (2006.01)
*D01F 6/14* (2006.01)     *A61L 15/24* (2006.01)
*A61L 15/60* (2006.01)

(86) Internationale Anmeldenummer:
**PCT/EP2014/002525**

(87) Internationale Veröffentlichungsnummer:
**WO 2015/078538 (04.06.2015 Gazette 2015/22)**

(54) **HYDROGELIERENDE FASERN SOWIE FASERGEBILDE**

HYDROGELLING FIBERS AND FIBER STRUCTURES

FIBRES HYDROGÉLIFIANTES ET PRODUIT À BASE DE FIBRES HYDROGÉLIFIANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.11.2013 DE 102013019888**

(43) Veröffentlichungstag der Anmeldung:
**05.10.2016 Patentblatt 2016/40**

(73) Patentinhaber: **Carl Freudenberg KG
69469 Weinheim (DE)**

(72) Erfinder:
• **DUNCKER-RAKOW, Samuel
64295 Darmstadt (DE)**
• **SCHLESSELMANN, Bernd
69469 Weinheim (DE)**
• **KRAMPFL, Katharina
74613 Öhringen (DE)**

(56) Entgegenhaltungen:
EP-A1- 2 650 413     WO-A1-2012/048768
JP-A- H03 279 410

• **AHMET ÇAY ET AL: "Properties of electrospun poly(vinyl alcohol) hydrogel nanofibers crosslinked with 1,2,3,4-butanetetracarboxylic acid", JOURNAL OF APPLIED POLYMER SCIENCE, Bd. 129, Nr. 6, 18. Februar 2013 (2013-02-18), Seiten 3140-3149, XP055073782, ISSN: 0021-8995, DOI: 10.1002/app.39036**

**Beschreibung**

Technisches Gebiet

Die vorliegende Erfindung betrifft hydrogelierende Fasern oder ein-, zwei- oder dreidimensionale Fasergebilde, hergestellt aus einem ersten Faserrohmaterial, wobei das erste Faserrohmaterial wasserlöslichen Polyvinylalkohol und/oder Polyvinylalkohol-Copolymer enthält, sowie ein dazugehöriges Herstellungsverfahren. Des Weiteren betrifft die Erfindung die Verwendung von derartigen Fasern oder Fasergebilden zur Wundversorgung, insbesondere in Produkten zur medizinischen Versorgung, wie Wundauflagen, sowie in Hygiene- und Kosmetikprodukten oder dergleichen. Die Erfindung betrifft ferner Produkte zur medizinischen Versorgung, insbesondere Wundauflagen, sowie Hygiene- und Kosmetikprodukte.

Die erfindungsgemäßen Fasern oder Fasergebilde können vorteilhafter Weise im direkten Kontakt zur Wunde oder zum Körper verwendet werden. Aus den erfindungsgemäßen Fasern oder Fasergebilden hergestellte Produkte für die Wundversorgung quellen mit wässrigen Lösungen oder Wundexsudat und bilden ein stabiles Hydrogel aus, welches eine außerordentlich hohe Höchstzugkraft und Höchstzugkraftdehnung besitzt. Dadurch lassen sich Wundauflagen, die die erfindungsgemäßen Fasern oder Fasergebilde enthalten, in einem Stück wieder von der Wunde entfernen. Zudem besitzen die erfindungsgemäßen Fasern oder Fasergebilde eine besonders hohe Aufnahmekapazität sowie ein besonders hohes Rückhaltevermögen (Retention) für wässrige Lösungen.

Stand der Technik

Aus der WO 01/30407 A1 ist ein Verfahren zur Herstellung von Hydrogelen zur Verwendung als Wundauflagen bekannt, mit dem Verbrennungen oder andere Hautverletzungen behandelt werden können. Im Zuge des Verfahrens wird eine wässrige Lösung von Polyvinylalkohol, Agar-Agar und zumindest eines weiteren natürlichen Polymers zubereitet. Diese Lösung wird bei 70-80°C in Einweg-Plastikgefäßen abgefüllt und versiegelt. Nach Abkühlung auf Raumtemperatur werden die in den Einweg-Plastikgefäßen abgefüllten Proben bestrahlt und damit sterilisiert.
In der WO 2005/103097 A1 sind Hydrogele beschrieben, die zumindest ein Polyvinylalkohol-Sternpolymer aufweisen. Dabei werden die Hydrogele durch wiederholtes Einfrieren und Auftauen einer wässrigen Lösung enthaltend zumindest ein Polyvinylalkohol- Sternpolymer und optional weitere Komponenten hergestellt. Des Weiteren können derartige Hydrogele durch Einwirkung ionisierender Strahlung auf eine wässrige Lösung enthaltend zumindest ein Polyvinylalkohol - Sternpolymer oder durch eine Reaktion eines Polyvinylalkohol - Sternpolymers in wässriger Lösung mit vernetzenden Reagenzien hergestellt werden. In JPH03279410 ist ein Verfahren zur Herstellung von wasserlöslichen Fasern aus Polyvinylalkohol beschrieben, in dem Fasern aus einer Polyvinylalkohollösung gesponnen, mit einer Säure behandelt und anschließend getrocknet werden. Die beschrieben Fasern sind schnell im Wasser löslich und geeignet als Bindemittelfasern in der Papierherstellung.

Aus Ahmet et al., "Properties of electrospun poly(vinyl alcohol) hydrogel nanofibers crosslinked with 1,2,3,4-butanetetracarboxylic acid", (JOURNAL OF APPLIED POLYMER SCIENCE, Bd. 129, Nr. 6, 18. Februar 2013, Seiten 3140-3149), ist bekannt nano-fasrige Hydrogele aus Polyvinylalkohol herzustellen welches mit einer 1,2,3,4-Butantetracarboxylsäure vernetzt sind.

Nachteilig an den derzeit bekannten Methoden zur Herstellung von Hydrogelen, insbesondere für die Wundbehandlung, ist die aufwändige Herstellungsweise und die problematische Weiterverarbeitung der Hydrogele, sowie gegebenenfalls das Auftreten von chemischen Verunreinigungen in den zum Beispiel durch eine chemische Reaktion vernetzten Hydrogelen. Zudem besitzen Hydrogel-Filme im Gegensatz zu Fasern und Fasergebilden eine kleinere Oberfläche, wodurch sie eine geringere Absorptionskapazität für Wasser oder wässrige Lösungen aufweisen. Besonders bei der Verwendung von Polyvinylalkohol als Rohmaterial für Hydrogele ist darauf zu achten, dass der Polyvinylalkohol einen hohen Vernetzungsgrad aufweist, da sich sonst keine Hydrogele, sondern Lösungen des Polyvinylalkohols in dem flüssigen Medium ausbilden. Wünschenswert ist demzufolge eine hohe Stabilität des Polyvinylalkohols gegenüber Wasser oder wässrigen Lösungen. Außerdem zeichnen sich gerade Polyvinylalkohol und Polyvinylalkohol-Copolymere durch eine hohe Biokompatibilität und Bioverträglichkeit aus, so dass ein zunehmender Bedarf an weiteren Ausführungsformen von Hydrogelen oder hydrogelierenden Materialien mit Polyvinylalkohol und/oder PolyvinylalkoholCopolymeren besteht, die zudem kostengünstig und einfach herzustellen sind und eine unproblematische Weiterverarbeitung ermöglichen.

In J Mater Sci (2010) 45:2456-2465 wird ein Verfahren zur Herstellung von Nanofasern und Fasergebilden aus Polyvinylalkohol mittels Elektrospinnen beschrieben, in dem die Fasern bzw. Fasergebilde mittels einer Temperaturbehandlung gegenüber wässrigen Lösungen stabilisiert werden. Nachteilig an Fasergebilden aus Nanofasern ist, dass diese aufgrund ihres Faserdurchmessers zwischen 244 bis 270nm eine sehr geringe Festigkeit und Höchstzugkraftdehnung sowie eine nur geringe Absorptionskapazität zeigen. Zudem sind die beschriebenen Fasern gegenüber wässrigen Lösungen stabilisiert, so dass sie keine gelierenden Eigenschaften besitzen, nicht in wässriger Lösung quellen sowie nicht geeignet sind Wasser in der Faser einzuschließen (fehlendes Rückhaltevermögen).

Wundauflagen aus hydrogelierenden Fasern z.B. aus Carboxymethylcellulose oder modifizierter Cellulose sind grundsätzlich bekannt. Diese bilden jedoch ein sehr weiches Hydrogel mit geringer Höchstzugkraft und Höchstzugkraftdehnung mit der Wundflüssigkeit aus. Nachteilig hieran ist, dass sie schlecht in einem Stück von der Wunde oder aus der Wundhöhle wieder entfernt werden können. So kann es passieren dass Reste der Wundauflage in der Wunde verbleiben, die aufwändig durch Reinigung der Wunde wieder entfernt werden müssen. Dies bedeutet einen erhöhten Zeit- und damit auch Kostenaufwand für das Krankenhauspersonal. Zudem kann die Wunde durch die Reinigung wieder verletzt oder geschädigt werden.

Fasern aus Polyvinylalkohol sind kommerziell in verschiedenen Typen erhältlich und umfassen Polyvinylalkohol unterschiedlicher Wasserlöslichkeit. Wasserunlösliche Typen der Polyvinylalkohole sind beispielsweise die sogenannten High Strength-Polyvinylalkohol-Fasern mit einer besonders hohen Höchstzugkraft im trockenen Zustand. Handelsübliche wasserlösliche Fasern aus Polyvinylalkohol sind mit einer temperaturabhängigen Wasserlöslichkeit z.B. einer Wasserlöslichkeit oberhalb einer Temperatur von 90°C, von 70°C, von 60°C, von 40°C oder 20°C erhältlich. Handelsübliche Fasern aus Polyvinylalkohol können zwar in ihrer Wasserlöslichkeit variieren, sie weisen aber keine hydrogelierenden Eigenschaften auf und zeigen somit auch kein Rückhaltevermögen für Wasser.

Aus der WO 2012/048768 sind Fasern und Fasergebilde, hergestellt aus wasserlöslichem Polyvinylalkohol bekannt, die durch Tempern von Fasern oder Fasergebilden aus einem ersten Faserrohmaterial umfassend wasserlöslichen Polyvinylalkohol und/oder wasserlösliches Polyvinylalkohol-Copolymer bei einer vorbestimmten Tempertemperatur vernetzt worden sind. Diese Fasern und Fasergebilde lassen sich bereits auf vergleichsweise einfachem und kostengünstigem Weg herstellen und unproblematisch weiterverarbeiten. Sie finden Anwendung beispielsweise als Wundverbände oder Wundauflagen. Diese zeichnen sich durch eine erhöhte Stabilität, insbesondere eine hohe Höchstzugkraft und Höchstzugkraftdehnung im hydrogeliertem Zustand aus, so dass sie in einem Stück wieder von der Wunde oder aus der Wundhöhle entfernt werden können. Praktische Versuche haben jedoch ergeben, dass bei diesem Verfahren vergleichsweise lange Temperdauern, beispielsweise von mehr als 4 h benötigt werden, um die Fasern oder Fasergebilde mit einer ausreichenden Stabilität zu versehen.

Darstellung der Erfindung

Die vorliegende Erfindung beschäftigt sich mit der Aufgabe das aus der WO 2012/048768 bekannte Verfahren dahingehend weiterzubilden, dass die Temperdauern verringert werden können. Die mit diesem Verfahren erhaltenen Fasern oder Fasergebilde sollen ferner eine unproblematische Weiterverarbeitung und/oder Anwendung ermöglichen. Zudem sollen aus den erfindungsgemäßen Fasern oder Fasergebilden hergestellte Wundverbände oder Wundauflagen eine hohe Stabilität, insbesondere eine hohe Höchstzugkraft und Höchstzugkraftdehnung im hydrogeliertem Zustand aufweisen, so dass sie in einem Stück wieder von der Wunde oder aus der Wundhöhle entfernt werden können.

Erfindungsgemäß wird diese Aufgabe durch die Gegenstände der unabhängigen Ansprüche gelöst. Vorteilhafte Ausführungsformen sind Gegenstand der abhängigen Ansprüche.

Überraschenderweise konnte festgestellt werden, dass die erforderliche Temperdauer und/oder Tempertemperatur in einem Verfahren der eingangs genannten Art deutlich reduziert werden kann, wenn die Fasern oder Fasergebilde vor dem Tempern mit einem Säurekatalysator versehen werden. So wurde gefunden, dass bei dieser Vorgehensweise Temperdauern von weniger als 0,5 h ausreichen, um die Fasern oder Fasergebilde mit einer ausreichenden Stabilität zu versehen. Praktische Versuche haben ergeben, dass mit Temperdauern von 1 Minute bis 0,5 h, vorzugsweise von 1 Minute bis 15 Minuten, noch bevorzugter von 1 Minute bis 10 Minuten, noch bevorzugter von 1 Minute bis 5 Minuten, und insbesondere von 1 Minute bis 3 Minuten besonders gute Ergebnisse erzielt werden können.

Die Tempertemperaturen liegen dabei im Bereich von 100 bis 210 °C, vorzugsweise von 130 bis 190 °C, und insbesondere von 150 bis 180°C. Dieser Effekt lässt sich vermutlich darauf zurückzuführen, dass die beim Tempern stattfindende Vernetzungsreaktion zumindest teilweise eine chemische Reaktion darstellt, die durch saure Katalyse beschleunigt werden kann.

Als Säurekatalysator können die verschiedensten Säuren eingesetzt werden. Vorzugsweise wird als Säurekatalysator eine Lewis-Säure und/oder eine Protonensäure verwendet. Dabei kann die Protonensäure eine oder mehrere Säurefunktionen aufweisen und bevorzugt eine organische Säure, noch bevorzugter eine $C_{1-10}$-Carbonsäure, insbesondere eine $C_{2-6}$-Carbonsäure sein. Die Carbonsäure kann verzweigt oder unverzweigt sein. Gemäß einer Ausführungsform der Erfindung liegt die Carbonsäure unsubstituiert vor. Gemäß einer alternativen Ausführungsform der Erfindung weist die Carbonsäure einen oder mehrere Substituenten auf. Bevorzugte Substituenten sind dabei Alkohol-, Amino- und/oder Halogenreste.

Vorteilhaft an der Verwendung niederer Carbonsäuren, beispielsweise der $C_{2-6}$-Carbonsäuren ist, dass diese leicht flüchtig sind und/oder leichtflüchtige thermische Zerfallsprodukte bilden und somit beim Tempern rückstandslos aus dem Substrat entfernt werden können. Zu diesem Zweck haben sich folgende Säuren als besonders geeignet erwiesen Essigsäure, Ameisensäure, Propionsäure und Zitronensäure.

In manchen Fällen kann es jedoch auch zweckmäßig sein nicht flüchtige Säuren einzusetzen, um hierdurch das Substrat mit einer gewünschten Eigenschaft auszustatten. Zu diesem Zweck haben sich folgende Säuren als besonders geeignet erwiesen: Benzoesäure, para-Toluolsulfonsäure. Durch Verwendung dieser Säuren kann das Produkt mit sauren Eigenschaften ausgestattet werden. Hierdurch kann beispielsweise eine pH Regulierung bei kosmetischen Anwendungen erzielt werden.

Als besonders geeignete Lewis-Säuren haben sich Säuren, ausgewählt aus der Gruppe bestehend aus zwei- oder dreiwertigen Metallionen, insbesondere Zn(II) und Al(III) erwiesen.

Im erfindungsgemäßen Verfahren können die Fasern oder Fasergebilde aus dem ersten Faserrohmaterial auf die verschiedenste Art und Weise mit dem Säurekatalysator versehen werden. Als zweckmäßig hat sich insbesondere erwiesen den Säurekatalysator aus einer Lösung oder Suspension aufzubringen. Die Lösung oder Suspension enthält dabei den Säurekatalysator sowie ein Lösungsmittel oder Lösungsmittelgemisch, welches zweckmäßigerweise so ausgewählt wird, dass die Fasern oder Fasergebilde keine oder nur eine geringe Löslichkeit in ihm haben. Vorzugsweise wird ein Lösungsmittel eingesetzt, in dem die Fasern oder Fasergebilde eine Löslichkeit bei 20 °C von weniger als 1 g pro Liter, vorzugsweise von 0 bis 0,6 g pro Liter, insbesondere von 0 bis 0,3 g pro Liter aufweisen. Als geeignet haben sich beispielsweise Lösungsmittel auf Basis von Alkohol, vorzugsweise ausgewählt aus der Gruppe bestehend aus Ethanol, Methanol, Isopropylalkohol erwiesen.

Die Lösung oder Suspension kann beispielsweise durch Foulardieren, Sprühen, Pflatschen und/oder Schaumimprägnierung auf die Fasern oder Fasergebilde aufgebracht werden. Als besonders geeignet hat sich das Aufbringen mittels Foulard erwiesen. Diese Aufbringungsart hat den Vorteil, dass behandelte Fasergebilde vollständig durchdrungen werden können.

Gemäß einer Ausführungsform der Erfindung wird das Lösungsmittel nach dem Aufbringen der den Säurekatalysator enthaltenden Lösung oder Suspension durch Trocknen, beispielsweise in einem der Auftragsapparatur nachgelagerten Ofen getrocknet. Dies hat den Vorteil, dass in nachgelagerten Verfahrensschritten keine Lösungsmittelabsaugung vorgesehen werden muss. Alternativ kann das Entfernen des Lösungsmittels auch simultan mit dem Tempern der Fasern bzw. des Fasergebildes erfolgen. Dies hat den Vorteil, dass die Anzahl der Verfahrensschritte reduziert werden kann.

Die auf die Fasern oder das Fasergebilde aufgebrachte Menge an Säurekatalysator beträgt vorteilhafterweise 0,01 bis 15 Gew.%, vorzugsweise 0,05 bis 10 Gew.%, insbesondere 0,1 bis 1 Gew.%, jeweils bezogen auf das Gewicht der Fasern.

Gemäß einer weiteren bevorzugten Ausführungsform der Erfindung wird vor dem Versehen mit dem Säurekatalysator, ein Verfestigungsverfahren zur Herstellung eines ein, zwei oder dreidimensionalen Fasergebildes, insbesondere zur Herstellung eines Vliesstoffs, angewendet. Diese Vorgehensweise ist vorteilhaft, da verfestigte Fasern deutlich einfacher mit einem Säurekatalysator ausgerüstet werden können. Nicht verfestigte Fasern sind dagegen nur schwer ausrüstbar, da sie aneinander haften, Klumpen bilden und deshalb nur schwer gleichmäßig beschichtet werden können.

Mit dem erfindungsgemäßen Verfahren können Fasern oder Fasergebilde, die wasserlösliches Polyvinylalkohol enthalten, so durch Tempern behandelt werden, dass sie mit wässrigen Lösungen oder Wundexsudat, insbesondere mit einer 0,9 prozentigen, wässrigen Natriumchlorid-Lösung (physiologische Kochsalzlösung) oder mit einer wässrigen Lösung gemäß der in DIN 13726-1 in Punkt 3.2.2.3 aufgeführten Prüflösung A ein stabiles Hydrogel ausbilden, welches eine sehr hohe Höchstzugkraft und Höchstzugkraftdehnung besitzt. Zudem weisen derartige Fasern oder Fasergebilde eine hohe Stabilität gegenüber Wasser oder wässrigen Lösungen auf. Ferner zeichnen sich die erfindungsgemäßen Fasern oder Fasergebilde durch eine hohe Aufnahmekapazität und ein hohes Rückhaltevermögen für Wasser oder wässrige Lösungen, insbesondere 0,9 prozentige, wässrige Natriumchlorid-Lösung (physiologische Kochsalzlösung) oder eine wässrige Lösung gemäß der in DIN 13726-1 in Punkt 3.2.2.3 aufgeführten Prüflösung A aus.

In einem weiteren Aspekt der Erfindung werden Fasern oder Fasergebilde, ein-, zwei-oder dreidimensional, vorgeschlagen, die mit dem oben beschriebenen Verfahren hergestellt werden. Diese Fasern oder Fasergebilde können hergestellt werden aus Fasern aus einem ersten Faserrohmaterial, wobei das erste Faserrohmaterial wasserlöslichen Polyvinylalkohol und/oder Polyvinylalkohol-Copolymer enthält und wobei das Faserrohmaterial durch Tempern bei einer vorbestimmten Tempertemperatur, die größer ist als die Glasübergangstemperatur und kleiner ist als die Schmelz- oder Zersetzungstemperatur des verwendeten ersten Faserrohmaterials eine vorbestimmte Temperdauer lang vernetzt und hydrogelierend ausgebildet wird. Durch diese Behandlung wird das Faserrohmaterial stabilisiert und insbesondere auch die Fasern oder Fasergebilde, die aus dem Faserrohmaterial hergestellt sind, gegenüber wässrigen Lösungen stabilisiert, so dass sie in wässriger Lösung eine deutlich verringerte Löslichkeit zeigen.

Dabei bilden die Fasern oder Fasergebilde gleichzeitig mit wässrigen Lösungen ein stabiles Hydrogel aus.

Unter Tempern im Sinne dieser Erfindung wird ein Prozess verstanden, bei dem das Faserrohmaterial, vorzugsweise in Form von Fasern oder Fasergebilden bei einer vorbestimmten Temperatur eine vorbestimmte Zeit, vorzugsweise bei Atmosphärendruck und Gasatmosphäre, insbesondere Luftatmosphäre, erhitzt werden. Zweckmäßigerweise wird das Faserrohmaterial in Form von Fasern oder eines Fasergebildes im trockenen Zustand, vorteilhafter Weise bei einer Restfeuchte von weniger als 10 Gew.%, noch bevorzugter von weniger als 5 Gew.%, noch bevorzugter von weniger als 3 Gew.% getempert. Zweckmäßigerweise werden die Fasern oder Fasergebilde zuerst auf die vorbestimmte Temperatur gebracht und dann die vorbestimmte Zeit lang auf dieser vorbestimmten Temperatur gehalten. Dabei können auftretende Temperaturschwankungen von zumindest +/-10%, insbesondere +/-5% und bevorzugt +/-1% toleriert werden. Zudem kann beim Temperprozess beliebig viel Luft zugeführt oder abgeführt werden und im Temperbereich die Luft auf verschiedene Weise (z.B. Umluft, Durchluft) umgewälzt werden. Beim Temperprozess können auch andere Prozessgase wie Stickstoff oder Sauerstoff zusätzlich zugeleitet werden, um den Temperprozess und damit die Eigenschaften der Fasern oder Fasergebilde in erwünschter Weise zu beeinflussen.

Besonders bevorzugt wird der Temperprozess im Falle der zweidimensionalen Fasergebilde oder Vliesstoffe mit Durchluft in einem Bandtrockner durchgeführt. Mit Hilfe der Durchluft kann die Temperdauer im Vergleich zu der Temperdauer mit reiner Umluft auf ein Vielfaches reduziert werden.

Vorteilhaft können durch das Tempern die Fasern oder Fasergebilde so vernetzt werden, dass sie gegenüber Wasser eine höhere Löslichkeitsstabilität aufweisen. Darüber hinaus erhalten die Fasern oder Fasergebilde durch das Tempern die Fähigkeit, mit Wasser oder wässrigen Lösungen, insbesondere mit 0,9 prozentiger Natriumchlorid-Lösung oder mit einer Lösung gemäß der in DIN 13726-1 in Punkt 3.2.2.3 aufgeführten Prüflösung A, ein stabiles Hydrogel auszubilden, welches sich durch eine besonders hohe Höchstzugkraft und Höchstzugkraftdehnung auszeichnet.

Zudem können durch das Tempern Verunreinigungen oder Rückstände, wie zum Beispiel Spinnhilfsmittel, Avivage, Lösungsmittel oder dergleichen, signifikant reduziert oder sogar bis auf eine Konzentration unterhalb der jeweiligen Nachweisgrenze reduziert werden. Des Weiteren weisen die erfindungsgemäßen Fasern oder Fasergebilde eine hohe Aufnahmekapazität und ein hohes Rückhaltevermögen (Retention) für Wasser, wässrige Lösungen, insbesondere für eine 0,9 Gew.%ige wässrige Natriumchlorid-Lösung oder für eine Lösung gemäß der in DIN 13726-1 in Punkt 3.2.2.3 aufgeführten Prüflösung A und/oder Wundexsudat auf.

So können die Fasern oder Fasergebilde für Wasser und/oder wässrige Lösungen eine Retention über 70 %, vorzugsweise von 70 % bis 100 % aufweisen. Im Fall von Fasern und/oder eindimensionalen, sowie zweidimensionalen Fasergebilden liegt bevorzugt eine relative Retention für 0,9 prozentige Natriumchlorid-Lösung oder für eine Lösung gemäß der in DIN 13726-1 in Punkt 3.2.2.3 aufgeführten Prüflösung A von über 70 % vor, noch bevorzugter über 80 %,- noch bevorzugter über 85 %, noch bevorzugter von 85 % bis 100%.

Zudem können die Fasern oder Fasergebilde eine relative Aufnahmekapazität für 0,9 prozentige Natriumchlorid-Lösung oder für eine Lösung gemäß der in DIN 13726-1 in Punkt 3.2.2.3 aufgeführten Prüflösung A von 4 bis 30 g/g aufweisen. Im Fall von Fasern und/oder eindimensionalen, sowie zweidimensionalen Fasergebilden liegt bevorzugt eine relative Aufnahmekapazität für 0,9 prozentige Natriumchlorid-Lösung oder für eine Lösung gemäß der in DIN 13726-1 in Punkt 3.2.2.3 aufgeführten Prüflösung A von 4 bis 30 g/g vor, besonders bevorzugt von 4 bis 25 g/g,- noch bevorzugter von 5 bis 20 g/g, noch bevorzugter von 7 bis 20 g/g. Somit lassen sich vorteilhaft toxikologisch unbedenkliche und biokompatible Fasern oder Fasergebilde, sowie daraus herstellbaren Gele, insbesondere Hydrogele, erzeugen.

Man versteht unter Fasern ein im Verhältnis zu seiner Länge dünnes und flexibles Gebilde. Fasern weisen einen geringen Durchmesser auf und können miteinander durch dementsprechende Verfestigungsverfahren zu Fasergebilden aufgebaut werden. Somit kann ein Fasergebilde mehrere Fasern aufweisen. Man kann zwischen ein-, zwei- und dreidimensionalen Fasergebilden unterscheiden. Ein eindimensionales Fasergebilde hat im Vergleich zu seiner Länge eine geringe Breite und eine geringe Höhe. Ein zweidimensionales Fasergebilde hat im Vergleich zu seiner Länge und Breite eine geringe Höhe. Unter dreidimensionalen Fasergebilden sind Fasergebilde zu verstehen, die mehrere Lagen von zweidimensionalen Fasergebilden aufweisen. Dabei können die einzelnen Lagen des dreidimensionalen Fasergebildes durch nachfolgend beschriebene Verfestigungsverfahren oder anderweitig zueinander verbunden werden.

Aus Polymeren lassen sich mittels des Trocken- oder des Nassspinnverfahrens Filamente herstellen und mittels des Spinnvliesverfahrens Spinnvliese. Die Filamente können dabei als eindimensionale Fasergebilde angesehen werden, während die Spinnvliese zweidimensionale Fasergebilde darstellen können. Durch Schneiden und/oder Kräuseln der Filamente können Stapelfasern hergestellt werden, die als eindimensionale Fasergebilde eingeordnet werden können. Durch Garndrehung können aus Stapelfasern Stapelfasergarne hergestellt werden. Sie können als eindimensionale Fasergebilde verstanden werden. Aus Filamenten aufgebaute Garne können aus einem Filament (Monofilamentgarn) oder mehreren Filamenten (Multifilamentgarn) ausgebildet sein. Sie können ebenfalls als eindimensionale Fasergebilde angesehen werden. Mischgarne können durch Garnspinnen mehr als einer unterschiedlichen Stapelfaser bzw. Naturfaser hergestellt werden. Garne wie Naturfasergarne, Stapelfasergarne oder Filamentgarne oder Mischgarne, können mittels textiltechnischer Verfahren wie Weben, Stricken, Wirken, Sticken, Legen oder Nähen z.B. zu Geweben, Gestricken, Gelegen oder Gewirken weiterverarbeitet werden. Die Gewebe, Gestricke, Gelege bzw. Gewirke können als zweidimensionale Fasergebilde angesehen werden. Mittels vliestechnischer Verfahren wie Krempeln oder dem Airlaidverfahren können aus Stapelfasern Stapelfaservliese oder Airlaidvliese hergestellt werden, die ebenfalls als zweidimensionale Fasergebilde angesehen werden können. Erfindungsgemäß bevorzugt werden wasserlösliche Stapelfasern verwendet, die mittels Krempeln zu einem Stapelfaservlies gelegt werden.

Unverfestigte Vliese, z.B. Stapelfaser- oder Spinnvliese, können durch Verfestigungsverfahren zu Vliesstoffen verfestigt werden. Es kann beispielsweise als Verfestigungsverfahren ein Kalandrieren angewendet werden. Dabei werden die unverfestigten Vliese zwischen Rollen geführt, wobei auf den Rollen angeordnete Verschweißflächen in den Vliesen zumindest teilweise die Vliese durchdringende Verschweißungen erzeugen. Werden punktförmige Verschweißungen erzeugt, dann wird das Verfestigungsverfahren als PS (point seal)-Verfestigungsverfahren bezeichnet. Es ist aber auch die Ausbildung von linienförmigen Verschweißungen oder vollflächigen Verschweißungen möglich. Als weiteres Verfestigungsverfahren kann eine Heißluftverfestigung im Durchlufttrockner angewendet werden, wobei bei diesem Verfahren Verfestigungen durch Verschmelzungen an den Berührungspunkten der Fasern erzeugt werden. Des Weiteren ist ebenfalls der Einsatz von Bindern oder Bindemitteln denkbar, wobei hierbei die Fasern über Brücken aus Bindern oder Bindemittel miteinander verbunden werden. Es können insbesondere auch mechanische Verfestigungsverfahren zum Einsatz kommen, wie z.B. das Nadelverfestigungsverfahren, bei dem die Verfestigung mittels Nadeln vorgenommen wird. Des Weiteren ist ebenfalls Walken oder Filzen oder dergleichen denkbar. Dabei kann auch eine Kombination von mehreren Verfestigungsverfahren angewendet werden. Bevorzugt werden das Nadelverfestigungsverfahren und/oder das PS-Verfestigungsverfahren angewendet.

Durch das Tempern können die wasserlöslichen Fasern aus Polyvinylalkohol oder die Fasergebilde, die die wasserlöslichen Fasern aus Polyvinylalkohol enthalten, vernetzt werden. Somit können sowohl die Fasern selbst als auch die Fasergebilde durch Tempern derartig verändert werden, dass sie eine höhere Stabilität gegenüber Wasser, insbesondere gegenüber einer 0,9 prozentigen, wässrigen Natriumchlorid-Lösung oder gegenüber einer Lösung gemäß der in DIN 13726-1 in Punkt 3.2.2.3 aufgeführten Prüflösung A aufweisen

Die getemperten Fasern oder die daraus hergestellten Fasergebilde weisen vorzugsweise einen löslichen Anteil von 1% bis 30%, vorzugsweise von 1% bis 25%, noch bevorzugter von 1% bis 20% und noch bevorzugter von 1% bis 15% in 0,9% iger, wässriger Natriumchlorid-Lösung oder in einer Lösung gemäß der in DIN 13726-1 in Punkt 3.2.2.3 aufgeführten Prüflösung A auf.

Darüber hinaus wird den Fasern oder dem Fasergebilde durch das Tempern in vorteilhafter Weise die Eigenschaft verliehen mit Wasser bzw. den oben genannten Lösungen ein stabiles Hydrogel mit hoher Höchstzugkraft und Höchstzugkraftdehnung auszubilden. Unter "hydrogelierend" ist die Fähigkeit zur Ausbildung eines Hydrogels zu verstehen, das als flüssige Phase Wasser oder eine wässrige Lösung, besonders bevorzugt eine 0,9 prozentige, wässrige Natriumchlorid-Lösung oder eine Lösung gemäß der in DIN 13726-1 in Punkt 3.2.2.3 aufgeführten Prüflösung A, aufweist.

Ein Hydrogel ist ein in Wasser gequollenes, hydrophiles polymeres Netzwerk. Insbesondere ist unter einem Hydrogel ein System aus mindestens einer festen und einer flüssigen Phase zu verstehen, wobei die feste Phase ein dreidimensionales Netzwerk ausbildet, dessen Poren durch wässrige Lösung ausgefüllt werden können und dadurch quellen. Beide Phasen können sich dabei vollständig durchdringen und demzufolge kann ein Gel im Vergleich zu einem Schwamm eine flüssige Phase stabiler gegenüber z.B. Druck speichern. Darüber hinaus besitzt ein Hydrogel ein hohes Rückhaltevermögen (Retention) für wässrige Lösungen.

Erfindungsgemäße Fasern oder Fasergebilde sind hydrogelierend ausgebildet und weisen demzufolge ein hervorragendes Bindungs- und Rückhaltevermögen für wässrige Phasen auf. Sie werden bevorzugt trocken auf die Wunde aufgelegt oder Wundhöhlen damit ausgefüllt. Sie bilden mit dem Wundexsudat stabile Hydrogele aus und schaffen somit ein optimales Wundklima zur Wundheilung ohne mit der Wunde zu verkleben. Eine derartige feuchte Wundbehandlung kann den Heilungsprozess unterstützen. Durch die hohe Höchstzugkraft und Höchstzugkraftdehnung des mit dem Wundexsudat ausgebildeten Hydrogels, lassen sich die Fasern oder Fasergebilde in einem Stück aus der Wunde oder Wundhöhle entfernen.

Ebenfalls für die feuchte Wundbehandlung können die erfindungsgemäßen Fasern oder Fasergebilde mit einer flüssigen Phase bestückt in hydrogelierter Form eingesetzt werden. Bevorzugt wird dabei als flüssige Phase Wasser verwendet und besonders bevorzugt eine 0,9 prozentige, wässrige Natriumchlorid-Lösung, Ringer-Lösung oder wirkstoffhaltige Lösungen oder eine Lösung gemäß der in DIN 13726-1 in Punkt 3.2.2.3 aufgeführten Prüflösung A.

Polyvinylalkohole sind Polymere und können durch Hydrolyse aus Polyvinylacetat hergestellt werden. Die technischen Eigenschaften des Polyvinylalkohols, wie insbesondere seine Wasserlöslichkeit hängen unter anderem vom Herstellungsverfahren, von der Molmasse und dem verbleibenden Anteil an Acetyl-Gruppen ab (Hydrolysegrad). Mit abnehmender Molmasse und Hydrolysegrad nimmt die Löslichkeit in Wasser zu. Je nach Molmasse und Hydrolysegrad besitzen die Polyvinylalkohole eine unterschiedliche Wasserlöslichkeit. So lösen sich einige Typen von Polyvinylalkohol erst bei einer erhöhten Temperatur in Wasser auf (z.B. ab 90°C). Fasern aus Polyvinylalkohol werden bei ihrer Herstellung üblicherweise auf ein Mehrfaches ihrer ursprünglichen Länge verstreckt und können hierbei auch erhitzt werden (Verstreckungstemperatur), um die Kristallinität und Festigkeit der Fasern zu erhöhen. Dabei werden durch Parallelorientierung der Molekülketten die Ausbildung von intermolekularen Wasserstoffbrückenbindungen ermöglicht. Damit kann auch die Wasserlöslichkeit der Polyvinylalkohol-Fasern eingestellt werden.

Die als erstes Faserrohmaterial verwendeten ungetemperten Fasern aus Polyvinylalkohol können erfindungsgemäß in einem Überschuss von Wasser bereits unterhalb einer Temperatur von 50°C, bevorzugt unterhalb 40°C, besonders bevorzugt unterhalb 30°C, noch bevorzugter unterhalb von 25°C wasserlöslich sein, wobei die ungetemperten Fasern selbstverständlich auch oberhalb dieser Werte wasserlöslich sein können. Ferner können die ungetemperten Fasern auch oberhalb von 15°C und/oder oberhalb von 20°C wasserlöslich sein. Insbesondere können die ungetemperten Fasern in einem Bereich zwischen 0°C und 150 °C oder zwischen 5°C und 100 °C oder zwischen 10°C und 100 °C, oder zwischen 15°C und 100 °C oder zwischen 20°C und 100 °C wasserlöslich sein, wobei unter wasserlöslich zu verstehen ist, dass sich die Fasern in einem Überschuss von Wasser zumindest zu 70 %, vorzugsweise zu mehr als 80 %, noch bevorzugter zu mehr als 90 % und insbesondere zu mehr als 95 %, und insbesondere zu 100 % auflösen.

Das Polyvinylalkohol, das für die Herstellung der Fasern aus Polyvinylalkohol verwendet wird, kann dabei durch Copolymerisation mit anderen Monomeren (z.B. Polyethylen-Vinylalkohol) oder durch den Einbau von funktionellen Gruppen modifiziert sein, wodurch weitere physikalische wie auch chemische Eigenschaften gegebenenfalls gezielt in die Fasern eingebaut werden. So ist im Fall der Verwendung von beispielsweise Polyethylen-Vinylalkohol die Anzahl an OH-Gruppen reduziert ausgebildet.

Bevorzugt können als Polyvinylalkohol-Copolymere Polyethylen-Vinylalkohol, Polyvinylalkohol-Styrol, Polyvinylalkohol-Vinylacetat, Polyvinylalkohol-Vinylpyrrolidon, Polyvinylalkohol-ethylenglykol und/oder Polyvinylalkohol, besonders bevorzugt Polyethylen-Vinylalkohol, Polyvinylalkohol-Vinylacetat, Polyvinylalkohol-Vinylpyrrolidon, Polyvinylalkohol-Vinylamin, Polyvinylalkohol-Acrylat, Polyvinylalkohol-Acrylamid, Polyvinylalkohol-ethylenglykol eingesetzt werden. Die Polyvinylalkohol-Copolymere können als Blockcopolymere und/oder Pfropfcopolymere und/oder Block- Pfropfcopolymere, statistische oder alternierende Systeme und jegliche Mischungen untereinander vorliegen. Der Anteil an anderen Monomereinheiten im Polyvinylalkohol beträgt dabei maximal 30Gew.%, vorzugsweise 1 bis 30 %, noch bevorzugter 5 bis 15 %, jeweils bezogen auf jeweils bezogen auf die Gesamtanzahl an Monomereinheiten im Polyvinylalkohol-Copolymer.

Es können aber auch andere funktionelle Gruppen in das Polvinylalkohol und/oder in die Fasern oder in das Fasergebilde z.B. durch Substitution oder polymeranaloge Reaktionen eingebracht werden. Als funktionelle Gruppen kommen hierbei insbesondere Carbonsäuren, ungesättigte Carbonsäuren wie Methyacrylsäuren, Acrylsäuren, Peroxycarbonsäuren, Sulfonsäuren, Carbonsäureester, Sulfonsäureester, Aldehyde, Thioaldehyde, Ketone, Thioketone, Amine, Ether, Thioether, Isocyanate, Thiocyanate, Nitrogruppen in Betracht. Der Anteil an anderen funktionellen Gruppen im Polyvinylalkohol beträgt dabei maximal 30 %, vorzugsweise 1 bis 30 %, noch bevorzugter 5 bis 15 %, jeweils bezogen auf die Anzahl an OH-Gruppen im Polyvinylalkohol.

Des Weiteren kann das erste Faserrohmaterial als physikalische Mischung zwischen dem wasserlöslichen Polyvinylalkohol und mindestens einem anderem Polymer (Polymer-Blend) ausgebildet sein. Der Anteil an wasserlöslichem Polyvinylalkohol beträgt dabei im Polymer-Blend mindestens 70 Gew.%, bezogen auf die Gesamtmasse des Polymer-Blends.

Vorteilhaft weist der daraus resultierende Polymer-Blend im Vergleich zu den eingesetzten Polymeren unterschiedliche physikalische Eigenschaften und gegebenenfalls auch chemische Eigenschaften auf. Üblicherweise sind dabei die Eigenschaften des Polymer-Blends eine Summe der Eigenschaften der verwendeten Polymere. Somit kann durch den Einsatz von Polymer-Blends eine Auswahl an ersten Faserrohmaterialien weiter vergrößert werden. Dabei können zur Ausbildung eines derartigen Polymer-Blends gelierende weitere Polymere, wie z.B. Alginate, Celluloseether, wie Carboxymethylcellulosen, Methyl-, Ethylcellulosen, Hydroxymethylcellulosen, Hydroxyethylcellulosen, Hydroxyalkylmethylcellulosen, Hydroxypropylcellulosen, Celluloseester, wie Celluloseacetat, oxidierte Cellulosen, bakterielle Cellulosen, Cellulosecarbonate, Gelatinen, Kollagene, Stärken, Hyaluronsäuren, Pektine, Agar, Polyacrylate, Polyvinylamine, Polyvinylacetate, Polyethylenglycole, Polyethylenoxide, Polyvinylpyrrolidone, Polyurethane oder nicht-gelierende weitere Polymere, wie z.B. Polyolefine, Cellulose, Cellulosederivate, regenerierte Cellulose wie Viskose, Polyamide, Polyacrylnitrile, Polyvinylchloride, Chitosane, Polylactide, Polyglykolide, Polyesteramide, Polcaprolactone, Polyhexamethylenterephthalate, Polyhydroxybutyrate, Polyhydroxyvalerate oder Polyester eingesetzt und zu dem wasserlöslichen Polyvinylalkohol hinzu gemischt werden. Die oben aufgeführten Blends können als Homopolymere oder Copolymere eingesetzt werden. Es können auch Blockcopolymere und/oder Pfropfcopolymere und/oder Block-Pfropfcopolymere, statistische oder alternierende Systeme und jegliche Mischungen untereinander eingesetzt werden.

Unter Alginate versteht man die Salze der Alginsäure, einem natürlichen in Algen vorkommenden Polymer, der beiden Uronsäuren $\alpha$-L-Guluronsäure und $\beta$-D-Mannuronsäure, die 1,4-glycosidisch verknüpft sind. Dabei wird von dem Begriff Aiginat E401, E402, E403, E404 und E405 (PGA) umfasst. Von dem Begriff Polyolefine wird PE, PB, PIB und PP umfasst. Von dem Begriff Polyamiden werden PA6, PA6.6, PA6/6.6, PA6.10, PA6.12 PA69, PA612, PA11, PA12, PA46, PA1212 und PA6/12 umfasst. Von dem Begriff Cellulose wird auch regenerierte Cellulose wie Viskose, sowie Cellulosederivate und chemisch und/oder physikalisch modifizierte Cellulose umfasst. Von dem Begriff Polyester werden PBT, BC, PET, PEN und UP umfasst.

Das Polyvinylalkohol, das für die Herstellung der Fasern aus Polyvinylalkohol verwendet wird oder aus dem die Polyvinylalkohol-Fasern bestehen, kann mit verschiedenen Hydrolysegraden und mittleren Molmassen zum Einsatz kommen.

Der Hydrolysegrad des Polyvinylalkohols beträgt insbesondere mehr als 70%, bevorzugt oberhalb von 75%, noch bevorzugter oberhalb von 80% und bis zu 100 %.

Das Massenmittel der Molmasse des Polyvinylalkohols liegt insbesondere im Bereich von 20000 bis 200000 g/mol, bevorzugt im Bereich von 30000 bis 170000 g/mol, besonders bevorzugt im Bereich von 40000 bis 150000 g/mol, noch bevorzugter im Bereich von 50000 bis 140000 g/mol, noch bevorzugter im Bereich von 70000 bis 120000 g/mol.

Das Zahlennmittel der Molmasse des Polyvinylalkohols liegt insbesondere im Bereich von 10000 bis 120000 g/mol, bevorzugt im Bereich von 20000 bis 100000 g/mol, besonders bevorzugt im Bereich von 20000 bis 80000 g/mol, noch bevorzugter im Bereich von 25000 bis 70000 g/mol.

Dabei können Fasern aus einem ersten Faserrohmaterial mit einem Fasertiter von 0,5 bis 12 dtex eingesetzt werden. Bevorzugt werden sie mit einem Fasertiter von 1 bis 8 dtex eingesetzt, besonders bevorzugt mit einem Fasertiter von 1,4 bis 7 dtex und noch bevorzugter mit einem Fasertiter von 1,4 bis 4 dtex. Dabei ist unter dtex oder Dezitex das Gewicht in Gramm der Faser bei einer ggf. theoretischen Länge von 10.000 m zu verstehen. Fasern mit einem Einzeltiter von kleiner als 0,5 dtex sind weniger geeignet.

Die Fasern aus einem ersten Faserrohmaterial können eine Länge von 30 bis 100 mm aufweisen. Bevorzugt werden sie mit einer Länge von 30 bis 90 mm eingesetzt, besonders bevorzugt mit einer Länge von 30 bis 80 mm und noch bevorzugter mit einer Länge von 35 bis 70 mm.

Insbesondere handelt es sich bei den Fasern aus dem ersten Faserrohmaterial um sogenannte Stapelfasern, die für die Herstellung von Stapelfaservliesstoffen verwendet werden.

Außerdem können die Fasern oder Fasergebilde zusätzlich weitere Fasern aus zumindest einem zweiten Faserrohmaterial aufweisen. Dabei kann das zweite Faserrohmaterial nicht-gelierend oder gelierend ausgebildet sein. Somit können als weitere Fasern nicht-gelierende oder gelierende Fasern eingesetzt werden.

Vorteilhaft kann durch den Einsatz von weiteren Fasern ein gewünschtes Verhalten der Fasern oder der Fasergebilde gezielt verbessert werden. So kann durch den Einsatz der weiteren Fasern die Aufnahmekapazität der Fasergebilde noch weiter erhöht und der Schrumpf des Fasergebildes in wässriger Lösung reduziert werden.

Als weiteres Faserrohmaterial für die weiteren Fasern kann Polyester, wie Polyethylenterephthalat, wasserunlösliches Polyvinylalkohol, wasserlösliches Polyvinylalkohol, das oberhalb einer Temperatur von 50°C wasserlöslich ist, Polyolefine, wie Polyethylen oder Polypropylen, Cellulose, Cellulosederivate, regenerierte Cellulose, wie Viskose, Polyamide, Polyacrylnitrile, Chitosane, Elastane, Polyvinylchloride, Polylactide, Polyglykolide, Polyesteramide, Polycaprolactone, pflanzliche Naturfasern, Alginate, modifiziertes Chitosan, Celluloseether, wie Carboxymethylcellulosen, Methyl-, Ethylcellulosen, Hydroxymethylcellulosen, Hydroxyethylcellulosen, Hydroxyalkylmethylcellulosen, Hydroxypropylcellulosen, Celluloseester, wie Celluloseacetat, oxidierte Cellulosen, bakterielle Cellulosen, Cellulosecarbonate, Gelatinen, Kollagene, Stärken, Hyaluronsäuren, Pektine, Agar, Polyvinylamine, Polyvinylacetate, Polyethylenglycole, Polyethylenoxide, Polyvinylpyrrolidone, Polyurethane und/oder Polyacrylate eingesetzt werden. Die aufgeführten zweiten Faserrohmaterialien können sowohl als Homopolymere als auch als Copolymere eingesetzt werden. Es können auch Blockcopolymere und/oder Pfropfcopolymere und /oder Block-Pfropfcopolymere, statistische oder alternierende Systeme und jegliche Mischungen untereinander eingesetzt werden.

Es ist auch der gleichzeitige Einsatz von gelierenden und nicht-gelierenden weiteren Fasern oder von Mischungen aus verschiedenen weiteren Fasern möglich. Bevorzugt ist dabei die Verwendung von weiteren Fasern aus Polyamid, Polyester, wasserunlöslichem Polyvinylalkohol oder Polyvinylalkohol, welches sich oberhalb einer Temperatur von 50°C löst, Polyacrylat, Polyacrylsäure und noch bevorzugter aus Polyester oder wasserunlösliches Polyvinylalkohol oder Polyvinylalkohol, welches sich oberhalb einer Temperatur von 50°C löst und/oder Mischungen hiervon.

Die weiteren Fasern können auch aus einem als Polymer-Blend ausgebildeten zweiten Faserrohmaterial hergestellt sein. Dabei ergeben sich für die weiteren Fasern die schon vorhergehend bei dem ersten Faserrohmaterial aufgezeigten Vorteile.

Die Fasern aus dem erstem Faserrohmaterial oder aus dem weiteren Faserrohmaterial können auch in Form einer Bikomponentenfaser und/oder Mehrkomponentenfaser eingesetzt werden. Dabei können die Bikomponentenfasern und/oder Mehrkomponentenfasern in geometrischen Formen wie "Core shell", "Side-by-Side", "Pie- oder Orange-type", "Matrix with fibrils" vorliegen.

Die Bikomponentenfasern und/oder Mehrkomponentenfasern des weiteren Faserrohmaterials können zur thermischen Verfestigung der Vliese genutzt werden. Bei Erwärmung dieser Fasern erfolgt eine thermische Bindung des Vlieses. Beispielsweise schmilzt bei einer Core shell-Faser der shell-Anteil und verfestigt somit das Vlies. Als Bikomponentenfasern und/oder Mehrkomponentenfasern können Fasern aus dem weiteren Faserrohmaterial aus Polyethylen/Polypropylen, Polyethylen/Polyester, CoPolyester/Polyethylenterephthalat, Polyamid6/ Polyamid6.6, Polybutylenterephthalat/Polyethylenterephthalat eingesetzt werden.

Vorteilhaft kann durch die Verwendung von weiteren Fasern die Aufnahmekapazität von Wasser, insbesondere von einer 0,9 prozentigen Natriumchlorid-Lösung oder von einer Lösung gemäß der in DIN 13726-1 in Punkt 3.2.2.3 aufgeführten Prüflösung A, im Vergleich zu Fasergebilden ohne weitere Fasern deutlich erhöht werden, da insbesondere mittels der nicht-gelierenden Fasern ein Gel-Blocking-Effekt, der ab einer vorbestimmten Sättigung eine weitere Aufnahme von Wasser, insbesondere von einer 0,9 prozentigen Natriumchlorid-Lösung oder von einer Lösung gemäß der in DIN 13726-1 in Punkt 3.2.2.3 aufgeführten Prüflösung A, verhindert, verringert werden kann. Zudem kann der Schrumpf der Fasergebilde, die Fasern aus dem erstem Faserrohmaterial enthalten, in wässriger Lösung durch Beimengen von weiteren Fasern deutlich verringert werden.

Dabei kann der Schrumpf von zumindest zweidimensionalen Fasergebilden durch Ausstanzen von 10,0 cm x 10,0 cm (Fläche1) große Stücken und Eintauchen derselben in eine 0,9 %ige wässrige Natriumchlorid-Lösung oder eine Lösung gemäß der in DIN 13726-1 in Punkt 3.2.2.3 aufgeführten Prüflösung A bestimmt werden. Die ausgestanzten und getränkten Stücke werden aus der Lösung entnommen und für 2min abgetropft. Danach wird die Größe der Stücke abgemessen (Fläche 2). Der Schrumpf der Vliesstoffe kann dann nach folgender Formel berechnet werden:

$$Schrumpf\ [\%] = 100 - \frac{Fläche2\ [cm^2]}{Fläche1\ [cm^2]} * 100$$

Der Anteil an weiteren Fasern in den Fasergebilden kann 1 bis 70 Gew.% betragen. Bevorzugt beträgt der Anteil 1 bis 65 Gew.%, besonders bevorzugt 5 bis 60 Gew.%, noch bevorzugter 10 bis 50 Gew.%, noch bevorzugter zwischen 15 und 40 Gew.%.

Die weiteren Fasern können einen Fasertiter von 0,5 bis 12 dtex aufweisen. Bevorzugt werden sie mit einen Fasertiter von 1 bis 8 dtex eingesetzt, besonders bevorzugt mit einen Fasertiter von 1,4 bis 7 dtex und noch bevorzugter mit einen Fasertiter von 1,4 bis 4 dtex. Dabei ist unter dtex oder Dezitex das Gewicht in Gramm der Faser bei einer ggf. theoretischen Länge von 10.000 m zu verstehen. Fasern mit einem Einzeltiter von kleiner als 0,5 dtex sind weniger geeignet.

Die weiteren Fasern können eine Länge von 30 bis 100 mm aufweisen. Bevorzugt werden sie mit einer Länge von 30 bis 90 mm eingesetzt, besonders bevorzugt mit einer Länge von 30 bis 80 mm und noch bevorzugter mit einer Länge von 35 bis 70 mm.

Insbesondere handelt es sich bei den weiteren Fasern aus dem weiterem Faserrohmaterial um sogenannte Stapelfasern, die für die Herstellung von Stapelfaservliesstoffen verwendet werden.

Des Weiteren können die Fasern oder die Fasergebilde zusätzlich Additive aufweisen. Dabei können als Additive pharmakologische Wirkstoffe oder Medikamente, wie Antibiotika, Analgetika, Antiinfektiva, entzündungshemmende Mittel, wundheilungsfördernde Mittel oder dergleichen, antimikrobielle, antibakterielle oder antivirale Agentien, blutstillende Mittel, Enzyme, Aminosäuren, Antioxidantien, Peptide und/oder Peptidsequenzen, Polysaccharide (z.B. Chitosan), Wachstumsfaktoren (z.B. Purine, Pyrimidine), lebende Zellen, Tricalciumphosphat, Hydroxyapatit, insbesondere speziell Hydroxyapatitnanopartikel, geruchsadsorbierende Additive wie Aktivkohle, Cyclodextrine, Metalle wie Silber, Gold, Kupfer, Zink, Kohlenstoffverbindungen, wie Aktivkohle, Graphit oder dergleichen, kosmetische Wirkstoffe, Vitamine und/oder Verarbeitungshilfsstoffe wie oberflächenaktive Substanzen, Netzmittel, Avivagen, Antistatika eingesetzt werden

Durch den Einsatz von zumindest einem Additiv können die Fasern oder Fasergebilde zudem vorteilhaft mit weiteren physikalischen, chemischen sowie biologischen Eigenschaften ausgestattet werden. So ermöglicht beispielsweise eine Ausrüstung der Fasern oder Fasergebilde mit Silber oder Silbersalzen oder antimikrobiellen Agentien wie Polyhexanid (Polyhexamethylenbiguanid), Chlorhexidin, Cetylpyridiniumchlorid, Benzalkoniumchlorid, Medihoney, PVPlod, Wasserstoffperoxid, 8-Chinolinol, Chloramin, Ethacridinlactat, Nitrofural,oder Octenidin (N-Octyl-1-[10-(4-octyliminopyridin-1-yl)decyl]pyridin-4-imin) eine antibakterielle Wirkung der Fasern oder Fasergebilde.

Beispielsweise können die Fasern oder Fasergebilde mit einer ethanolischen Lösung, die ein antimikrobielles Agens enthält, ausgerüstet werden. Bevorzugt werden die Fasern oder Fasergebilde mit einer ethanolischen Lösung, die ein antimikrobielles Agens wie Polyhexanid, Octenidin oder Silbersalze enthält, mittels eines Foulards ausgerüstet. Es kommen jedoch auch jegliche andere Beschichtungsverfahren in Betracht. Zudem können die Fasern oder Fasergebilde mit einer wässrigen Lösung, die das antimikrobielle Agens enthält, ausgerüstet werden. Bevorzugt wird bei dem Auftrag aus wässriger Lösung eine kontrollierte Menge an Wasser eingesetzt, unter der die Fasern oder Fasergebilde nicht irreversibel hydrogelieren und sich in ihrer morphologischen Struktur verändern. Insbesondere kommen hier Beschichtungsverfahren wie der Schaumauftrag, Kisscoater oder dergleichen in Betracht.

Die erfindungsgemäßen Fasergebilde können ein Flächengewicht gemessen nach DIN EN 29073 von 10 bis 1000 g/m$^2$ aufweisen. Im Fall von zweidimensionalen Fasergebilden liegt bevorzugt ein Flächengewicht von 10 bis 700 g/m$^2$ vor, besonders bevorzugt von 20 bis 600 g/m$^2$, noch bevorzugter von 50 bis 500 g/m$^2$, noch bevorzugter von 70 bis 450 g/m$^2$, noch bevorzugter von 80 bis 400 g/m$^2$, noch bevorzugter von 90 bis 350 g/m$^2$, noch bevorzugter von 100 bis 300 g/m$^2$, noch bevorzugter von 120 bis 240 g/m$^2$ vor.

Im Fall von zwei- oder dreidimensionalen Fasergebilden liegt die Dicke des Fasergebildes vorzugsweise im Bereich von 0,2 bis 10 mm, bevorzugt im Bereich von 0,5 bis 8 mm, noch bevorzugter im Bereich von 0,7 bis 7 mm, noch bevorzugter im Bereich von 0,8 mm bis 6 mm, noch bevorzugter im Bereich von 0,9 bis 5 mm, besonders bevorzugt im Bereich von 1,0 bis 4 mm.

Im Fall von zwei- oder dreidimensionalen Fasergebilden werden diese bevorzugt thermisch oder mechanisch verfestigt. Besonders bevorzugt werden diese mechanisch durch Vernadelung verfestigt. Dabei liegt die Einstichdichte bevorzugt im Bereich von 70 bis 200 Einstichen pro Quadratzentimeter, besonders bevorzugt im Bereich von 70 bis 170 Einstichen pro Quadratzentimeter, besonders bevorzugt im Bereich von 80 bis 150 Einstichen pro Quadratzentimeter, besonders bevorzugt im Bereich von 100 bis 150 Einstichen pro Quadratzentimeter.

Die erfindungsgemäßen Fasergebilde können eine besonders hohe Höchstzugkraft sowohl in Längs- als auch in Querrichtung des Fasergebildes im hydrogeliertem Zustand aufweisen. Beispielsweise weisen erfindungsgemäße Fasergebilde, die ein Flächengewicht von 140 bis 220 g/m$^2$ haben und mechanisch durch Vernadelung beispielsweise mit einer Einstichdichte von 100-150 Einstichen pro Quadratzentimeter verfestigt wurden, eine Höchstzugkraft im hydrogelierten Zustand von oberhalb 0,3 N/2cm auf. Die bevorzugte Höchstzugkraft im hydrogeliertem Zustand liegt oberhalb von 0,4 N/2cm, noch bevorzugter oberhalb von 0,5 N/2cm noch bevorzugter oberhalb von 0,8 N/2cm, noch bevorzugter oberhalb von 1,0 N/2cm, noch bevorzugter oberhalb von 1,5 N/2cm, noch bevorzugter oberhalb von 2,0 N/2cm und/oder unterhalb von 50 N/2cm, und/oder unterhalb von 40 N/2cm, und/oder unterhalb von 35 N/2cm. Dementsprechend liegt eine Höchstzugkraft im hydrogelierten Zustand bevorzugt im Bereich von 0,3 N/2cm bis 50 N/2cm, noch bevorzugter von 0,4 N/2cm bis 40 N/2cm, noch bevorzugter von 0,5 N/2cm bis 30 N/2cm, noch bevorzugter von 0,8 N/2cm bis 25 N/2cm, noch bevorzugter von 1 N/2cm bis 25 N/2cm, noch bevorzugter von 1,5 N/2cm bis 25 N/2cm, noch bevorzugter von 2 N/2cm bis 25 N/2cm.

Die erfindungsgemäßen Fasergebilde können eine besonders hohe Höchstzugkraftdehnung sowohl in Längs- als auch in Querrichtung des Fasergebildes im hydrogeliertem Zustand aufweisen. Die bevorzugte Höchstzugkraftdehnung im hydrogeliertem Zustand liegt bei 20 bis 300%, besonders bevorzugt bei 30 bis 250% noch bevorzugter bei 50 bis 200%, noch bevorzugter bei 70 bis 200%, noch bevorzugter bei 80 bis 200%, noch bevorzugter bei 90 bis 190%, noch bevorzugter bei 90 bis 180%. Beispielsweise weisen erfindungsgemäße Fasergebilde, die ein Flächengewicht von 140 bis 220 g/m$^2$ haben und mechanisch durch Vernadelung beispielsweise mit einer Einstichdichte von 100-150 Einstichen pro Quadratzentimeter verfestigt wurden, die oben genannten Höchstzugkraftdehnungswerte auf.

Wie oben beschrieben können die hydrogelierend ausgebildeten Fasern oder Fasergebilden hergestellt werden, in dem mit einem Säurekatalysator versehene Fasern oder Fasergebilde aus einem ersten wasserlöslichen Faserrohmaterial umfassend Polyvinylalkohol und/oder unsubstituiertes oder teilweise unsubstituiertes Polyvinylalkohol-Copolymer bei einer vorbestimmten Tempertemperatur, die größer als eine Glasübergangstemperatur und kleiner ist als eine Schmelztemperatur des verwendeten ersten Faserrohmaterials, eine vorbestimmte Temperdauer lang getempert werden, so dass die Fasern vernetzt werden.

Vorteilhaft können durch diesen sehr einfachen Prozess auf schnelle und rationelle Weise Fasern oder Fasergebilde hergestellt werden, die hydrogelierende Eigenschaften aufweisen. Dabei sind nur wenige Prozessschritte zur Stabilisierung der Fasern oder Fasergebilde notwendig.

Zudem können durch das Tempern gegebenenfalls in den Fasern oder Fasergebilde enthaltene Verunreinigungen, wie zum Beispiel Avivage, Spinnhilfsmittel oder Lösungsmittel, entfernt werden.

Die vorbestimmte Tempertemperatur wird vorzugsweise so gewählt, dass sie größer ist als die Glasübergangstemperatur des verwendeten ersten Faserrohmaterials. Zudem kann die vorbestimmte Tempertemperatur derart gewählt werden, dass sie kleiner ist, als die Schmelztemperatur des verwendeten ersten Faserrohmaterials. Werden dabei mehrere Fasern aus unterschiedlichem Faserrohmaterial verwendet, so wird die vorbestimmte Temperatur vorzugsweise so gewählt, dass sie unterhalb der Schmelztemperatur oder Zersetzungstemperatur bevorzugt aller verwendeten Faserrohmaterialien liegt.

In vielen Anwendungsfällen haben sich Tempertemperaturen in einem Temperaturbereich von 85 bis 220 °C, besonders bevorzugt von 100 bis 200 °C, noch bevorzugter von 120°C bis 190 °C, noch bevorzugter zwischen 130°C und 180°C, ganz besonders bevorzug zwischen 140°C und 180°C, noch bevorzugter zwischen 150°C und 175°C als zweckmäßig erwiesen.

Praktische Versuche haben ergeben, dass mit Temperdauern von 1 Minute bis 0,5 h, vorzugsweise von 1 Minute bis 15 Minuten, noch bevorzugter von 1 Minute bis 10 Minuten, noch bevorzugter von 1 Minute bis 5 Minuten, und insbesondere von 1 Minute bis 3 Minuten besonders gute Ergebnisse erzielt werden können.

Durch die Auswahl derartiger Tempertemperaturen und Temperzeiten kann das erfindungsgemäße Vernetzen der Fasern oder Fasergebilde besonders schonend für die Fasern oder Fasergebilde durchgeführt werden. Zudem können durch die Auswahl dieser Temperbedingungen die Eigenschaften der Fasern oder Fasergebilde optimal eingestellt werden. So besitzen die Fasern oder Fasergebilde durch Auswahl dieser Temperbedingungen eine hohe Absorptionskapazität und Rückhaltevermögen (Retention) sowie eine sehr hohe Höchstzugkraft und Höchstzugkraftdehnung im hydrogeliertem Zustand. Durch Variation der Tempertemperaturen und Temperzeiten ist eine unterschiedlich ausgebildete Vernetzung steuerbar, so dass die vernetzten Fasern oder Fasergebilde gegebenenfalls unterschiedliche Eigenschaften aufweisen. Auch können durch die Auswahl dieser Temperbedingungen aus den Fasern oder Fasergebilden ggf. vorhandene Verunreinigungen wie Lösungsmittelrückstände oder Faserhilfsmittel und Faserverarbeitungshilfsmittel wie Avivagen, Netzmittel, Antistatika sogar bis auf einen nicht mehr nachweisbaren Gehalt reduziert werden. Dies ist insbesondere für die Verwendung der Fasern oder Fasergebilde in Wundauflagen von Vorteil, da die oben genannten Verunreinigungen oder Faserhilfsmittel /-verarbeitungsmittel toxikologisch bedenklich sein können.

Insbesondere vor dem Tempern kann ein Verfahren angewendet werden, um ein-, zwei- oder dreidimensionale Fasergebilde zu erhalten. Dabei kann aus den Fasern z.B. mittels eines vorhergehend beschriebenen Verfahrens das jeweilige Fasergebilde hergestellt werden.

Vorteilhaft können durch ein derartiges Verfestigungsverfahren die Fasern oder Fasergebilde in eine gewünschte Form gebracht werden und in dieser Form verfestigt werden.

Zudem können auch weitere Fasern aus zumindest einem zweiten Faserrohmaterial zugemischt werden.

Des Weiteren kann eine Nachbehandlung durchgeführt werden. Zudem ist eine Zumischung von Verarbeitungshilfsstoffen, insbesondere vor dem Verfestigungsverfahren möglich. Ebenfalls eine Zugabe von z.B. zuvor beschriebenen Additiven kann vorgenommen werden.

Als mögliche Nachbehandlung kann eine Nachverfestigung, eine Sterilisierung wie z.B. Strahlensterilisation oder Sterilisation mit Ethylenoxid, eine Bestrahlung, eine Beschichtung, eine Ausrüstung, eine Applikation von Avivagen, eine chemische Modifizierung oder eine Weiterverarbeitung wie z.B. Rascheln, Einbringen von Verstärkungsfasern vorgenommen werden.

Eine besonders bevorzugte Nachbehandlung der Fasern oder Fasergebilde ist eine Plasmabehandlung, um insbesondere die Hydrophilie der Fasern oder Fasergebilde zu erhöhen. Plasma ist ein Gemisch aus neutralen und geladenen Teilchen. In speziellen Fällen liegen nur geladene Teilchen vor. Im Plasma liegen verschieden Spezies wie Elektronen, Kationen, Anionen, neutrale Atome, neutrale oder geladene Moleküle vor. Durch die im Plasma enthaltenen aktiven Teilchen können Oberflächen wie z.B. Fasern oder Vliesstoffe modifiziert werden. Dabei können verschiedene Effekte erzielt werden wie z.B. eine Veränderung der Oberfläche durch Plasmaätzen, Plasmaaktivierung oder Plasmapolymerisation. Bei der Plasmaaktivierung wird die Oberfläche durch ein Plasma unter Zusatz von Sauerstoff aktiviert. Bei der Plasmapolymerisation werden weitere organische Vorläuferverbindungen in die Prozesskammer gegeben.

Die Fasern oder Faserhilfsstoffe können durch das Tempern hydrophob ausgebildet werden, da durch das Tempern die Faserhilfmittel und Faserverarbeitungsmittel reduziert werden können. Die Plasmabehandlung kann sowohl unter Atmosphärendruck als auch unter Vakuum insbesondere unter Zusatz von Sauerstoff durchgeführt werden. Auch können weitere Substanzen wie Acrylsäure während der Plasmabehandlung zugeben werden.

Zudem ist eine bevorzugte Nachbehandlung die Sterilisation der Fasern oder Fasergebilde für die Anwendung insbesondere für Wundauflagen. Bevorzugt wird die Sterilisation durch Strahlensterilisation oder durch Sterilisation mit Ethylenoxid durchgeführt. Durch die Sterilisation können die Eigenschaften wie z.B. Absorptionskapazität und/oder Höchstzugkraft und Höchstzugkraftdehnung im hydrogelierten Zustand positiv beeinflusst werden.

Die einzelnen Verfahrensschritte, Tempern, Verfestigen, Zumischen von weiteren Fasern, Zugabe von Additiven, Zugabe von Verarbeitungshilfsstoffen, und Nachbehandeln können mehrfach in beliebiger Reihenfolge wiederholt werden. Als zweckmäßig hat sich erwiesen die Fasern oder die Fasergebilde zumindest einmal dabei bei einer vorbestimmten Tempertemperatur eine vorbestimmte Temperdauer lang zu tempern.

Als Verarbeitungshilfsstoffe können Avivage, antistatische Mittel, Tenside, Stabilisatoren, Gleitmittel oder dergleichen zum Einsatz kommen.

In einer bevorzugten Variante des Herstellungsverfahrens werden die Fasern aus einem ersten Faserrohmaterial, insbesondere wasserlösliche Polyvinylalkohol-Stapelfasern, zur Vernetzung bei einer vorbestimmten Tempertemperatur, die oberhalb der Glasübergangstemperatur und unterhalb der Schmelztemperatur der Fasern aus einem ersten Faserrohmaterial liegt, insbesondere 10 min bis 7h lang, getempert. Darauffolgend kann optional eine Zumischung von weiteren Fasern, insbesondere von nicht-gelierenden Fasern, besonders bevorzugt von Polyester-Fasern, mit einem Gewichts-Anteil von 10 bis 50 Gew.% vorgenommen werden. Dann kann aus den so hergestellten Fasern ein zweidimensionales Fasergebilde, wie zum Beispiel ein Vliesstoff, gegebenenfalls unter Einsatz von Verarbeitungshilfsstoffen, wie zum Beispiel Avivage oder antistatischen Mitteln, mittels eines Verfestigungsverfahrens hergestellt werden.

In einer anderen bevorzugten Variante des Herstellungsverfahrens können optional Fasern aus einem ersten Faserrohmaterial mit weiteren Fasern aus einem zweiten Faserrohmaterial vermischt werden, wobei der Anteil an weiteren Fasern bevorzugt 10-50 Gew.% beträgt. Es können aber auch nur Fasern aus einem ersten Faserrohmaterial verwendet werden. Bevorzugt werden als Fasern aus einem ersten Faserrohmaterial Polyvinylalkohol-Fasern und als weitere Fasern aus einem zweiten Faserrohmaterial Polyester-Fasern verwendet. Aus diesen Fasern kann mittels eines Verfestigungsverfahrens ein zweidimensionales Fasergebilde wie zum Beispiel ein Vliesstoff hergestellt werden. Nachfolgend kann das so hergestellte zweidimensionale Fasergebilde bei einer Tempertemperatur oberhalb der Glasübergangstemperatur und unterhalb der Schmelztemperatur der Fasern aus einem ersten Faserrohmaterial getempert werden. Optional kann ein derartig hergestelltes zweidimensionales Fasergebilde nachbehandelt werden.

In einem weiteren Aspekt der Erfindung wird die Verwendung von Fasern oder Fasergebilden, wie vorhergehend beschrieben, vorgeschlagen, wobei derartige Fasern oder Fasergebilde insbesondere zur Herstellung von Materialien für medizinische Anwendungen, insbesondere für Wundauflagen und Wundverbände angewendet wird und insbesondere für die Herstellung von Wundauflagen für den Bereich der modernen Wundversorgung.

Zudem können die Fasern oder Fasergebilde zur Herstellung von anderen Materialien für medizinische Anwendungen wie Nahtmaterialien, Implantate, Tissue Egineering Scaffolds, transdermale Patches, Drug-delivery Produkte, Trägermaterialien oder Ostomieprodukte angewendet werden.

Auch ist eine Verwendung zur Herstellung von Trägermaterialien, Dämmstoffen, Filtermaterialien zur Herstellung von Hygiene-, Kosmetik,-Haushaltsprodukten, technischen Absorberprodukten, wie Kabelummantelungen, Produkten für den Lebensmittelbereich, wie Lebensmittelverpackungen möglich. Als Hygieneprodukte können unter anderem Damenhygieneprodukte, Windeln und Inkontinenzprodukte verstanden werden. Von den Haushaltsprodukten werden ebenfalls Reinigungsmaterialien umfasst.

Für die jeweilige Verwendung ergeben sich unter anderem die vorhergehend genannten Vorteile.

Als weiterer Aspekt der Erfindung wird ein Wundverband oder eine Wundauflage, enthaltend Fasern oder Fasergebilde wie vorhergehend beschrieben, vorgeschlagen. Derartige Fasern oder Fasergebilde, können bevorzugt im Bereich der Modernen Wundversorgung, insbesondere zur modernen (feuchten) Wundbehandlung, eingesetzt werden.

Bei der modernen Wundversorgung stellen die Wundauflagen ein optimales feuchtes Wundklima ein, durch das die Wunde schneller abheilen kann. Die moderne Wundversorgung wird zur Behandlung von schwer heilenden Wunden wie chronischen Wunden verwendet, die z.B. durch Druckbeanspruchung oder Wundliegen (Dekubitus), Diabetes, Durchblutungsstörungen, Stoffwechselerkrankungen, Gefäßerkrankungen wie Veneninsuffizienz oder Immunschwäche entstehen können.

Die erfindungsgemäßen Fasern oder Fasergebilde besitzen zum einem eine hohe Absorptionskapazität für wässrige Lösungen und können somit das Wundexsudat absorbieren und einschließen. Durch die Aufnahme des Wundexsudats bilden die Fasern oder Fasergebilde zum anderen ein Hydrogel aus, welches die Flüssigkeit fest einschließt und auch unter Druck, der z.B. durch Anlegen eines Verbandes entsteht, zurückhält. Durch die Ausbildung des Hydrogels wird zudem ein feuchtes Wundklima geschaffen, durch das die Wundheilung gefördert wird. Die hydrogelierten Fasern oder Fasergebilde passen sich an die Struktur der Wundoberfläche an und können insbesondere auch für die Behandlung von Wundhöhlen verwendet werden. Durch die hohe Höchstzugkraft und Höchstzugkraftdehnung können die hydrogelierten Fasern oder Fasergebilde problemlos in einem Stück von der Wunde oder aus der Wundhöhle entfernt werden, ohne diese zu verletzen.

Solche Wundverbände oder Wundauflagen können auch analog klassischer Wundverbände oder Wundauflagen, wie beispielsweise Mullbinden, angewendet werden, weisen aber die vorteilhaften hydrogelierenden Eigenschaften auf, so dass durch erfindungsgemäße Wundverbände oder Wundauflagen ein vorteilhaft verbesserte Wundversorgung erreicht werden kann.

Ausführung der Erfindung

**Methoden und Messmethoden**

Im Folgenden wird dargelegt, wie verschiedene zur Charakterisierung der erfindungsgemäßen Fasern oder Fasergebilde verwendbare Parameter erfindungsgemäß zu bestimmen sind:

**1) Bestimmung der Dicke der zweidimensionalen Fasergebilde und / oder Vliesstoffs**

Gemäß DIN EN ISO 9073-2, jedoch ohne Konditionierung

**2) Bestimmung des Flächengewichtes der zweidimensionalen Fasergebilde und / oder Vliesstoffs**

Gemäß DIN EN 29073, jedoch ohne Konditionierung

**3) Bestimmung der Absorptionskapazität von Fasern**

Ein 600 mL Becherglas wird mit 300 mL 0,9%iger Natriumchlorid-Lösung (0,9 g Natriumchlorid in 100 mL destilliertem Wasser gelöst) oder mit einer Lösung gemäß der in DIN 13726-1 in Punkt 3.2.2.3 aufgeführten Prüflösung A gefüllt. Es werden 0,40 g (Fasergewicht trocken: $m_{trocken}$) der Fasern in die Lösung eingerührt. Die Fasern bleiben 10 min unter gelegentlichen Rühren mittels Glasstab im Becherglas. Die Zeiterfassung erfolgt mit einer Stoppuhr. Ein vortariertes Metallsieb (32 mesh) wird auf ein 2000 mL Becherglas gelegt. Der gesamte Inhalt des 600 mL Becherglases wird über das Metallsieb gegossen. Die Fasern werden 5 min im Metallsieb abgetropft. Das Gewicht des Metallsiebes einschließlich der Fasern wird ermittelt. Das Tara des Metallsiebes wird vom Gewicht abgezogen. Man erhält das Fasergewicht der hydrogelierten Fasern ($m_{nass}$).

Die Ermittlung der Absorptionskapazität der Fasern erfolgt mit folgender Formel:

$$\text{Relative } Absorptionskapazität \left[ {}^{g}\!/_{g} \right] = \frac{m_{nass} - m_{trocken}}{m_{trocken}}$$

Dabei ist

m$_{nass}$ die Masse aus der Messprobe und der absorbierten Flüssigkeit am Ende der Prüfung in g
m$_{trocken}$ die Masse der trockenen Messprobe in g

**4) Bestimmung der Absorptionskapazität von zweidimensionalen Fasergebilden oder Vliesstoffen in Anlehnung an DIN EN ISO 9073-6**

Die Prüfung der Absorptionskapazität erfolgt in Anlehnung an DIN EN ISO 9073-6; Absorption von Flüssigkeiten.
Als vorgegebene Flüssigkeit (Prüfmedium) laut Punkt 5.2.7 in der DIN EN ISO 9073-6 wird eine 0,9%ige Natriumchlorid-Lösung (0,9 g Natriumchlorid in 100 mL destilliertem Wasser) oder eine Prüflösung A gemäß der DIN 13726-1 Punkt 3.2.2.3 verwendet.
Das verwendete Prüfmedium wird bei dem jeweiligen Messergebnis mit angegeben.
Das Vorbereiten der Messproben (Größe von 10*10 cm) und Durchführung der Bestimmung erfolgt analog der DIN EN ISO 9073-6, jedoch ohne Konditionierung.
Die Absorptionskapazität wurde zudem abweichend von der Norm nach zwei verschiedenen Absorptionszeiten bestimmt:

1) Absorptionskapazität nach 1 min: gemäß Norm werden die Messproben 1 min in das Prüfmedium getaucht und 2 min abtropfen gelassen
2) Absorptionskapazität nach 1h: die Messproben werden 1h in das Prüfmedium getaucht und 2 min abtropfen gelassen

Die Absorption von Flüssigkeit (LAC) in Prozent berechnet sich laut DIN EN ISO 9073-6 nach folgender Formel:

$$LAC\ [\%] = \frac{m_n - m_k}{m_k} \times 100$$

Dabei ist

m$_k$ die Masse der trockenen Messprobe in g
m$_n$ die Masse aus der Messprobe und der absorbierten Flüssigkeit am Ende der Prüfung in g.

Die relative Absorption in g/g errechnet sich wie folgt:

$$Relative\ Absorption \left[ {}^{g}\!/_{g} \right] = \frac{m_n - m_k}{m_k}$$

Die absolute Absorption in g/m$^2$ errechnet sich wie folgt:

$$Absolute\ Absorption \left[ {}^{g}\!/_{m^2} \right] = Relative\ Absorption \left[ {}^{g}\!/_{g} \right] \times Flächengewicht\ [g/m^2]$$

Die hydrogelierten Messproben nach der Bestimmung der Absorptionskapazität nach 1h werden weiterverwendet für die Bestimmung des Rückhaltvermögens (Retention) von zweidimensionalen Fasergebilden und / oder Vliesstoffen (Punkt 5) und Bestimmung des löslichen Anteils von zweidimensionalen Fasergebilde und / oder Vliesstoffe (Punkt 6).

**5) Bestimmung des Rückhaltvermögens (Retention) von zweidimensionalen Fasergebilden oder Vliesstoffen**

Für die Bestimmung werden die hydrogelierten Messproben nach der Bestimmung der Absorptionskapazität (Punkt 4) nach 1h verwendet (Absorptionskapazität nach 1h); zudem werden die ermittelten Werte der Massen von den trockenen Messproben, die bei der Bestimmung der Absorptionskapazität ermittelt wurden, verwendet:

$m_k$ die Masse der trockenen Messprobe in g

Die Messproben werden jeweils auf einen flachen Metallnetz mit einer Größe von 15 x 15 cm gelegt, welches über einer Schüssel platziert ist, so dass Flüssigkeit von der Messprobe in die Schüssel ablaufen kann. Die Messprobe wird mit einem Gewicht, welches flächig einen Druck von 40 mmHg auf die gesamte Fläche der Messprobe ausübt (dies entspricht einem Gewicht von 5,434 kg auf einer Fläche von 100 cm$^2$) über einen Zeitraum von 2 min beaufschlagt. Danach wird das Gewicht der Messprobe genau abgewogen ($M_{Druck}$)

Das relative Rückhaltevermögen (Retention) in g/g errechnet sich wie folgt:

$$Relatives\ R\ddot{u}ckhalteverm\ddot{o}gen\ (Retention)\left[\frac{g}{g}\right] = \frac{m_{Druck} - m_k}{m_k}$$

Das Rückhaltevermögen (Retention) in Prozent errechnet sich wie folgt:

$$R\ddot{u}ckhalteverm\ddot{o}gen\ (Retention)[\%] = \frac{Relatives\ R\ddot{u}ckhalteverm\ddot{o}gen}{Relative\ Absorption\ nach\ 1h} * 100$$

### 6) Bestimmung des löslichen Anteils von zweidimensionalen Fasergebilden oder Vliesstoffen

Für die Bestimmung werden die hydrogelierten Messproben nach der Bestimmung der Absorptionskapazität (Punkt 4) nach 1h verwendet (Absorptionskapazität nach 1h); zudem werden die ermittelten Werte der Massen von den trockenen Messproben, die bei der Bestimmung der Absorptionskapazität ermittelt wurden, verwendet:

$m_k$ die Masse der trockenen Messprobe in g

Die hydrogelierte Messprobe wird in ein tariertes 100 mL Becherglas ($m_{Becherglas}$) gelegt. Das Becherglas mit Messprobe wird in einen handelsüblichen Labortrockenschrank mit Umluft mit einer Temperatur von 70°C gestellt und die hydrogelierte Messprobe dadurch getrocknet. Nach 24h wird das Becherglas mit der getrockneten Messprobe aus dem Trockenschrank heraus genommen. Nach Abkühlen wird das Gewicht der Messprobe ($m_{trocken}$) bestimmt, wobei das Becherglas zusammen mit der Messprobe ($m_{gesamt}$) ausgewogen wird und das Gewicht des Becherglases von dem Gewicht abgezogen wird:

$$m_{trocken} = m_{gesamt} - m_{Becherglas}$$

Der lösliche Anteil in Prozent wird wie folgt berechnet:

$$L\ddot{o}slicher\ Anteil\ [\%] = 100 - (\frac{m_{trocken}}{m_k} * 100)$$

### 7) Bestimmung des Schrumpfes von zweidimensionalen Fasergebilden oder Vliesstoffen

Der Schrumpf wird durch Ausstanzen von 10,0 cm x 10,0 cm (Fläche1) großen Stücken und Eintauchen derselben in ein Prüfmedium bestimmt. Das Prüfmedium ist entweder eine 0,9 %ige wässrige Natriumchlorid-Lösung oder in eine Prüflösung A gemäß DIN 13726-1 Punkt 3.2.2.3. Das jeweilige Prüfmedium wird bei dem Messergebnis mit angegeben. Die ausgestanzten und getränkten Stücke werden nach 1h aus der Lösung entnommen und für 2min abgetropft. Danach wird die Größe der Stücke abgemessen (Fläche 2). Der Schrumpf der Vliesstoffe kann dann nach folgender Formel berechnet werden:

$$\text{Schrumpf } [\%] = 100 - \left(\frac{\text{Fläche2 } [cm^2]}{\text{Fläche1 } [cm^2]}\right) * 100$$

**8) Bestimmung der Höchstzugkraft und der Höchstzugkraftdehnung bei maximaler Zugkraft von zweidimensionalen Fasergebilden und / oder Vliesstoffen im hydrogeliertem Zustand**

Für die Bestimmung werden DIN A4 große Vliesstoffstücke ausgestanzt und in einen Überschuss von 0,9%iger Natriumchlorid-Lösung oder Prüflösung A gemäß DIN 13726-1 Punkt 3.2.2.3 gelegt. Die Vliesstoffstücke werden nach 1h aus der Lösung entnommen. Mit Hilfe eines Stanzeisen werden aus den hydrogelierten Vliesstoff-Stücken die Messproben sowohl in Längsrichtung (Maschinenrichtung) des Vliesstoffes als auch in Querrichtung des Vliesstoffes gestanzt.

Das Stanzeisen zum Ausstanzen der Messprobe hat eine Länge von 90 mm. Die Breite beträgt am oberen und unteren Ende 35 mm. Nach 20 mm verengt sich das Stanzeisen jeweils an beiden Enden auf 20 mm (siehe Bild 1). Die Bestimmung der Höchstzugkraft und Höchstzugkraftdehnung erfolgt dann gemäß EN 29073-03 mit einer Zwick Z 1.0, jedoch mit folgenden Abweichungen

- Keine Konditionierung
- Abzugsgeschwindigkeit 200 mm/min
- Anderes Stanzeisen (wie oben beschrieben); Einspannlänge angepasst an die Stanzeisenlänge
- Andere Probenvorbereitung: die Proben werden nicht im trockenen Zustand, sondern im hydrogeliertem Zustand vermessen (Erstellung der Messproben wie oben beschrieben)

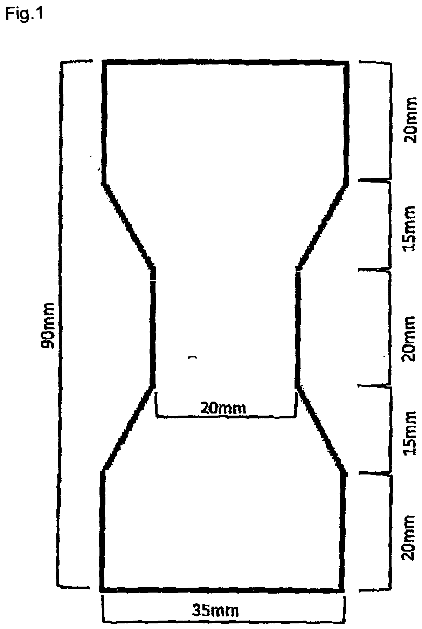

In Fig 1 ist das Stanzeisen zum Ausstanzen der Messproben dargestellt

**9) Bestimmung der Löslichkeit von wasserlöslichen Fasern**

Ein 250 mL Becherglas wird mit 200 mL destilliertem Wasser gefüllt und mit einer Heizplatte auf Prüftemperatur (Temperatur, bei der die Fasern aus Polyvinylalkohol wasserlöslich sind). Temperaturkontrolle erfolgt durch ein Thermometer.

Es werden jeweils 0,4 g der Fasern in die 200 mL des temperierten Wassers kurz eingerührt. Die Fasern bleiben zunächst 3 min ohne Rühren im Becherglas. Danach wird der Becherglasinhalt 7 min kräftig gerührt. Die Zeiterfassung erfolgt jeweils mit der Stoppuhr. Abschließend erfolgt eine optische Prüfung (mit dem Auge), ob sich die Fasern vollständig gelöst haben. Eine 100 prozentige Wasserlöslichkeit liegt dann vor, wenn keine festen Fasern oder Faserbestandteile mehr in der Lösung sichtbar sind.

**10) Bestimmung der Thermodesorption**

Bei der Bestimmung der Thermodesorption werden durch Aufheizen einer Probe von Fasern oder Fasergebilden bei 150°C für 20 min in den Fasern enthaltene organische Komponenten freigesetzt, mittels Kryotrap fokussiert und danach mittels Kaltaufgabesystem in die GC/MS injiziert. Dabei wird ein Thermodesorptionssystem GERSTEL und ein Kaltaufgabesystem KAS GERSTEL verwendet. Mittels GC/MS werden die freigesetzten Komponenten detektiert. Dabei wird ein GC Agilent Technologies 6890N Network GC System, Massenselektiver Detektor Agilent Technologies 5973 verwendet.

**11) Bestimmung der Netzzeit von zweidimensionalen Fasergebilden oder Vliesstoffen**

Es wird die Zeitdauer gemessen, die 1 Tropfen destilliertes Wasser braucht, um in das Fasergebilde oder den Vliesstoff einzusinken. Die Prüfung wird insgesamt mit 5 Tropfen durchgeführt und der Mittelwert gebildet.

**12) Untersuchung der Fasern oder Fasergebilde mittels XPS**

Die Messungen mittels XPS (X-ray Photoelectron Spectroscopy) wurden mit einem SSX-100 Spektrometer (Fa. SSI, US) mit monoenergetischer Al K$\alpha$1,2 Anregung (1486,6 eV) im Ultrahochvakuum (10-9 Torr) durchgeführt. Die Informationstiefe liegt zwischen 6 und 10 nm. Die Ladungskompensation für nichtleitende Proben wird mittels Floodgun erzielt. Vor Messbeginn werden die Proben über Nacht vakuumgelagert.

**Beispiele**

**Vergleichsbeispiel 1: Nadelverfestigte Vliesstoffe aus wasserlöslichen Polyvinylalkohol-Fasern mit anschließender thermischer Vernetzung**

Es wird ein nadelverfestigter Vliesstoff aus wasserlöslichen Polyvinylalkohol-Stapelfasern hergestellt. Die Polyvinylalkohol-Fasern sind bei einer Temperatur unterhalb von 25°C wasserlöslich und besitzen einen Fasertiter von 1,7 oder 2,2 dtex bei einer Stapelfaserlänge von 38 bzw. 51 mm. Die Polyvinylalkohol-Fasern werden durch eine Krempel zu einem Vlies gelegt und anschließend durch Vernadelung mit einer Einstichdichte von 100-170 Einstichen pro Quadratcentimeter vernadelt. Die nadelverfestigten Polyvinylalkohol-Vliesstoffe werden bei einer Temperatur von 150°C getempert, um eine Stabilisierung des Polyivnylalkohols zu erzielen. Die Vliesstoffe werden dabei in einem handelsüblichen Labortrockenschrank mit Umluft getempert. Ab einer Temperdauer von 2h stellt sich eine Stabilität der Polyvinylalkohol-Vliesstoffe ein, die sich durch die Ausbildung von stabilen, hydrogelierenden Vliesen in 0,9 %iger wässriger Natriumchlorid-Lösung oder Prüflösung A gemäß DIN 13726-1 Punkt 3.2.2.3 zeigt. Die Stabilität der Vliesstoffe steigt mit der Temperdauer. Bei einer Temperdauer von 2,5 bis 7h weisen die Vliesstoffe eine hohe Stabilität auf. Der lösliche Anteil der Vliesstoffe liegt nach 1h in Prüflösung A bei maximal 20%. Nach dem Tempern wird die relative Absorptionskapazität nach 1 min und 1h mit Prüflösung A als Prüfmedium bestimmt. Die relative Absorptionskapazität nach 1 min beträgt zwischen 5 und 20 g/g. Die relative Absorptionskapazität nach 1 h beträgt zwischen 5 und 20 g/g. Weiterhin wurde das Rückhaltevermögen (Retention) der Vliesstoffe nach 1h in Prüflösung A bestimmt. Diese beträgt zwischen 80-100%. Zudem wird der Schrumpf der verfestigten Vliesstoffe in Prüflösung A nach 1h in Prüflösung A bestimmt. Der Schrumpf der Polyvinylalkohol-Vliesstoffe beträgt abhängig von der Temperdauer und damit dem Vernetzungsgrad der Vliesstoffe zwischen 30 und 60%.

Tabelle 1: Beispiel für einen nadelverfestigten Vliesstoff aus wasserlöslichen Polyvinylalkohol-Fasern, der getempert wurde.

| Parameter | Beschreibung / Ergebnis |
|---|---|
| Polyvinylalkohol-Fasern | 1,5 bis 2,2 dtex, 40-70 mm |
| Temperatur, bei der die Polyvinylalkohol-Fasern wasserlöslich sind | Unterhalb von 25°C |
| Anteil an Polyvinylalkohol-Fasern [%] | 100 |
| Temperdauer bei 150°C [min] | 150-300 |
| Verfestigungsart | Vernadelung |
| Einstichdichte [#/cm$^2$] | 100-170 |
| Flächengewicht [g/m$^2$] | 150-210 |
| Dicke [mm] | 1,5-3,0 |
| Relative Absorptionskapazität [g/g] nach 1 min in Prüflösung A | 5,0-20,0 |
| Relative Absorptionskapazität [g/g] nach 1h in Prüflösung A | 5,0-20,0 |
| Rückhaltevermögen (Retention) [%] nach 1h in Prüflösung A | 80-100 |
| Löslicher Anteil nach 1h in Prüflösung A [%] | 0-20 |
| Schrumpf [%] | 30-50 |
| Höchstzugkraft im hydrogeliertem Zustand [N/2cm]; längs | 1-20 |
| Höchstzugkraftdehnung im hydrogeliertem Zustand [%]; längs | 80-300 |
| Höchstzugkraft im hydrogeliertem Zustand [N/2cm]; quer | 1-20 |
| Höchstzugkraftdehnung im hydrogeliertem Zustand [%]; quer | 80-300 |

**Beispiel 2: Nadelverfestigte Vliesstoffe aus wasserlöslichen Polyvinylalkohol-Fasern mit anschließender Säurebehandlung und thermischer Vernetzung**

Es wird ein nadelverfestigter Vliesstoff aus wasserlöslichen Polyvinylalkohol-Stapelfasern hergestellt. Die Polyvinylalkohol-Fasern sind bei einer Temperatur unterhalb von 25°C wasserlöslich und besitzen einen Fasertiter von 1,7 oder 2,2 dtex bei einer Stapelfaserlänge von 38 bzw. 51 mm. Die Polyvinylalkohol-Fasern werden durch eine Krempel zu einem Vlies gelegt und anschließend durch Vernadelung mit einer Einstichdichte von 100-170 Einstichen pro Quadratcentimeter vernadelt. Die nadelverfestigten Polyvinylalkohol-Vliesstoffe werden in einem Foulard-Bad mit einer Lösung von 1 % Gewicht Zitronensäure in Ethanol durchtränkt und bei Raumtemperatur in einem Abzug getrocknet. Die mit Zitronensäure beschichteten Polyvinylalkohol-Vliesstoffe werden bei einer Temperatur von 150°C getempert, um eine Stabilisierung des Polyivnylalkohols zu erzielen. Die Vliesstoffe werden dabei in einem handelsüblichen Labortrockenschrank mit Umluft getempert. Ab einer Temperdauer von 10 min stellt sich eine Stabilität der Polyvinylalkohol-Vliesstoffe ein, die sich durch die Ausbildung von stabilen, hydrogelierenden Vliesen in 0,9 %iger wässriger Natriumchlorid-Lösung oder Prüflösung A gemäß DIN 13726-1 Punkt 3.2.2.3 zeigt. Die Stabilität der Vliesstoffe steigt mit der Temperdauer. Bei einer Temperdauer von 30 min weisen die Vliesstoffe eine hohe Stabilität auf. Der lösliche Anteil der Vliesstoffe liegt nach 1h in Prüflösung A bei maximal 20%. Nach dem Tempern wird die relative Absorptionskapazität nach 1 min und 1h mit Prüflösung A als Prüfmedium bestimmt. Die relative Absorptionskapazität nach 1 min beträgt zwischen 5 und 20 g/g. Die relative Absorptionskapazität nach 1 h beträgt zwischen 5 und 20 g/g. Weiterhin wurde das Rückhaltevermögen (Retention) der Vliesstoffe nach 1h in Prüflösung A bestimmt. Diese beträgt zwischen 80-100%. Zudem wird der Schrumpf der verfestigten Vliesstoffe in Prüflösung A nach 1h in Prüflösung A bestimmt. Der Schrumpf der Polyvinylalkohol-Vliesstoffe beträgt abhängig von der Temperdauer und damit dem Vernetzungsgrad der Vliesstoffe zwischen 30 und 60%.

Tabelle 2: Beispiel für einen nadelverfestigten Vliesstoff aus wasserlöslichen Polyvinylalkohol-Fasern, der getempert wurde

| Parameter | Beschreibung / Ergebnis |
|---|---|
| Polyvinylalkohol-Fasern | 1,5 bis 2,2 dtex, 40-70 mm |
| Temperatur, bei der die Polyvinylalkohol-Fasern wasserlöslich sind | Unterhalb von 25°C |
| Anteil an Polyvinylalkohol-Fasern [%] | 100 |
| Temperdauer bei 150°C [min] | 150-300 |
| Verfestigungsart | Vernadelung |
| Einstichdichte [#/cm$^2$] | 100-170 |
| Flächengewicht [g/m$^2$] | 150-210 |
| Dicke [mm] | 1,5-3,0 |
| Relative Absorptionskapazität [g/g] nach 1 min in Prüflösung A | 5,0-20,0 |
| Relative Absorptionskapazität [g/g] nach 1h in Prüflösung A | 5,0-20,0 |
| Rückhaltevermögen (Retention) [%] nach 1h in Prüflösung A | 80-100 |
| Löslicher Anteil nach 1h in Prüflösung A [%] | 0-20 |
| Schrumpf [%] | 30-50 |
| Höchstzugkraft im hydrogeliertem Zustand [N/2cm]; längs | 1-20 |
| Höchstzugkraftdehnung im hydrogeliertem Zustand [%]; längs | 80-300 |
| Höchstzugkraft im hydrogeliertem Zustand [N/2cm]; quer | 1-20 |
| Höchstzugkraftdehnung im hydrogeliertem Zustand [%]; quer | 80-300 |

Wie ein Vergleich der Beispiele 1 und 2 zeigt, führt die Vorbehandlung des Vliesstoffes mit Zitronensäure als Katalysator zu einer deutlichen Verringerung der Temperdauer wobei die guten mechanischen und physikalischen Eigenschaften des Vliesstoffs im hydrogelierten Zustand erhalten bleiben.

**Patentansprüche**

1. Verfahren zur Herstellung von hydrogelierend ausgebildeten Fasern oder Fasergebilden, in dem Fasern oder Fasergebilde aus einem ersten Faserrohmaterial umfassend wasserlöslichen Polyvinylalkohol und/oder wasserlösliches Polyvinylalkohol-Copolymer bei einer vorbestimmten Tempertemperatur von 100 bis 210 °C, die größer ist als eine Glasübergangstemperatur und kleiner ist als eine Schmelztemperatur des verwendeten ersten Faserrohmaterials eine vorbestimmte Temperdauer lang getempert werden, so dass die Fasern vernetzt werden, **dadurch gekennzeichnet, dass** die Fasern oder Fasergebilde vor dem Tempern mit einem Säurekatalysator versehen werden.

2. Verfahren nach Anspruch 1, **dadurch gekennzeichnet, dass** der Säurekatalysator eine Lewis-Säure und/oder eine Protonensäure, vorzugsweise eine organische Säure, noch bevorzugter eine $C_{1-10}$-Carbonsäure, insbesondere eine $C_{2-6}$-Carbonsäure ist.

3. Verfahren nach Anspruch 2, **dadurch gekennzeichnet, dass** die Protonensäure ausgewählt ist aus der Gruppe bestehend aus Essigsäure, Ameisensäure, Propionsäure, Zitronensäure, Benzoesäure, para-Toluolsulfonsäure und/oder die Lewis-Säure ausgewählt ist aus der Gruppe bestehend aus zwei- oder dreiwertigen Metallionen, insbesondere Zn (II) und Al(III)

4. Verfahren nach einem oder mehreren der Ansprüche 1 bis 3,
**dadurch gekennzeichnet, dass** das Versehen der Fasern oder Fasergebilde aus dem ersten Faserrohmaterial mit dem Säurekatalysator mittels einer Lösung oder Suspension des Säurekatalysators in einem Lösungsmittel, insbesondere ausgewählt aus Ethanol, Methanol oder Isopropanol durchgeführt wird.

5. Verfahren nach Anspruch 4, **dadurch gekennzeichnet, dass** die den Säurekatalysator enthaltende Lösung oder Suspension durch Foulardieren, Sprühen, Pflatschen und/oder Schaumimprägnierung auf die Fasern oder Fasergebilde aufgebracht wird.

6. Verfahren nach einem oder mehreren der Ansprüche 1 bis 5,
**dadurch gekennzeichnet, dass** eine Lösung oder Suspension eingesetzt wird, die den Säurekatalysator in einer Menge von 0,01 bis 10 Gew.%, vorzugsweise von 0,05 bis 5 Gew.%, insbesondere von 0,1 bis 1 Gew.%, jeweils bezogen auf das Gesamtgewicht des Lösungsmittels enthält.

7. Verfahren nach einem oder mehreren der Ansprüche 1 bis 6,
**dadurch gekennzeichnet, dass** die auf die Fasern oder das Fasergebilde aufgebrachte Menge an Säurekatalysator 0,01 bis 15 Gew.%, vorzugsweise 0,05 bis 10 Gew.%, insbesondere 0,1 bis 1 Gew.%, jeweils bezogen auf das Gewicht der Fasern.

8. Verfahren nach einem oder mehreren der Ansprüche 1 bis 7,
**dadurch gekennzeichnet, dass** das Lösungsmittel nach dem Aufbringen der den Säurekatalysator enthaltenden Lösung oder Suspension durch Trocknen entfernt wird.

9. Verfahren nach einem oder mehreren der Ansprüche 1 bis 8,
**dadurch gekennzeichnet, dass** die vorbestimmte Temperdauer 1 Minute bis 0,5 h, vorzugsweise von 1 Minute bis 15 Minuten, noch bevorzugter von 1 Minute bis 10 Minuten, noch bevorzugter von 1 Minute bis 5 Minuten, und insbesondere von 1 Minute bis 3 Minuten beträgt.

10. Verfahren nach einem oder mehreren der Ansprüche 1 bis 9,
**dadurch gekennzeichnet, dass** vor dem Versehen mit dem Säurekatalysator, ein Verfestigungsverfahren zur Herstellung eines zwei oder dreidimensionalen Fasergebildes, insbesondere zur Herstellung eines Vliesstoffs, angewendet wird.

11. Verfahren nach einem oder mehreren der Ansprüche 1 bis 10,
**dadurch gekennzeichnet, dass** weitere Fasern aus zumindest einem zweiten Faserrohmaterial zugemischt werden.

12. Hydrogelierend ausgebildete Fasern oder Fasergebilde, ein-, zwei-, oder dreidimensional, hergestellt gemäß einem Verfahren nach einem oder mehreren der Ansprüche 1 bis 11, wobei eine unter den Bedingungen des Temperns nichtflüchtige Säure als Säurekatalysator eingesetzt wurde.

13. Verwendung von Fasern oder Fasergebilden nach Anspruch 12 zur Herstellung von Materialien für medizinische Anwendungen, von Wundauflagen, Wundverbänden, Nahtmaterialien, Implantaten, Tissue Engineering Scaffolds, Arzneimitteln, Trägermaterialien, Dämmstoffen, Filtermaterialien, technische Absorberprodukten, Produkten für den Lebensmittelbereich, Hygieneprodukten, Kosmetikprodukten, Haushaltsprodukten, wobei als Hygieneprodukte Damenhygieneprodukte, Windeln und Inkontinenzprodukte umfasst sind, wobei als Haushaltsprodukte Reinigungsmaterialien umfasst sind.

14. Wundverbände oder Wundauflagen enthaltend Fasern oder Fasergebilde nach Anspruch 12.

**Claims**

1. Method for producing fibres or fibrous structures designed to hydrogel, in which method fibres or fibrous structures made from a first fibrous raw material comprising water-soluble polyvinyl alcohol and/or water-soluble polyvinyl alcohol copolymer are heat-treated for a predetermined heat-treatment time at a predetermined heat-treatment temperature of from 100 to 210°C, which is greater than a glass transition temperature and is less than a melting temperature of the first fibrous raw material used, with the result that the fibres are cross-linked, **characterized in that** the fibres or fibrous structures are provided with an acid catalyst before the heat-treatment.

2. Method according to Claim 1, **characterized in that** the acid catalyst is a Lewis acid and/or a protic acid, preferably an organic acid, yet more preferably a $C_{1-10}$-carboxylic acid, more particularly a $C_{2-6}$-carboxylic acid.

3. Method according to Claim 2, **characterized in that** the protic acid is selected from the group consisting of acetic acid, formic acid, propionic acid, citric acid, benzoic acid, paratoluenesulfonic acid and/or the Lewis acid is selected from the group consisting of divalent or trivalent metal ions, more particularly Zn(II) and Al (III) .

4. Method according to one or more of Claims 1 to 3, **characterized in that** the provision of the fibres or fibrous structures made from the first fibrous raw material with the acid catalyst is carried out by means of a solution or suspension of the acid catalyst in a solvent, selected especially from ethanol, methanol or isopropanol.

5. Method according to Claim 4, **characterized in that** the solution or suspension containing the acid catalyst is applied to the fibres or fibrous structures by padding, spraying, slop-padding and/or foam impregnation.

6. Method according to one or more of Claims 1 to 5, **characterized in that** a solution or suspension containing the acid catalyst in an amount of from 0.01 to 10% by weight, preferably from 0.05 to 5% by weight, more particularly from 0.1 to 1% by weight, based in each case on the total weight of the solvent, is used.

7. Method according to one or more of Claims 1 to 6, **characterized in that** the amount of acid catalyst applied to the fibres or the fibrous structure from 0.01 to 15% by weight, preferably from 0.05 to 10% by weight, more particularly from 0.1 to 1% by weight, based in each case on the weight of the fibres.

8. Method according to one or more of Claims 1 to 7, **characterized in that** the solvent is removed by drying after the application of the solution or suspension containing the acid catalyst.

9. Method according to one or more of Claims 1 to 8, **characterized in that** the predetermined heat-treatment time is from 1 minute to 0.5 h, preferably from 1 minute to 15 minutes, yet more preferably from 1 minute to 10 minutes, yet more preferably from 1 minute to 5 minutes, and more particularly from 1 minute to 3 minutes.

10. Method according to one or more of Claims 1 to 9, **characterized in that,** before the provision with the acid catalyst, a consolidation method is applied to produce a two-dimensional or three-dimensional fibrous structure, more particularly to produce a nonwoven.

11. Method according to one or more of Claims 1 to 10, **characterized in that** further fibres made from at least a second fibrous raw material are added.

12. Fibres or fibrous structures - one-dimensional, two-dimensional or three-dimensional - designed to hydrogel, produced in accordance with a method according to one or more of Claims 1 to 11, wherein the acid catalyst used was an acid which is non-volatile under the heat-treatment conditions.

**13.** Use of fibres or fibrous structures according to Claim 12 for the production of materials for medical applications, of wound dressings, wound bandages, suture materials, implants, tissue engineering scaffolds, medicaments, support materials, insulating materials, filter materials, technical adsorbent products, products for the food sector, hygiene products, cosmetic products, household products, wherein hygiene products encompass feminine hygiene products, nappies and incontinence products, wherein household products encompass cleaning materials.

**14.** Wound bandages or wound dressings containing fibres or fibrous structures according to Claim 12.

**Revendications**

**1.** Procédé de fabrication de fibres ou de structures de fibres hydrogélifiantes, selon lequel des fibres ou des structures de fibres en une première matière première fibreuse comprenant de l'alcool polyvinylique soluble dans l'eau et/ou un copolymère d'alcool polyvinylique soluble dans l'eau sont étuvées pendant une durée d'étuvage prédéterminée à une température d'étuvage prédéterminée de 100 à 210 °C, qui est supérieure à une température de transition vitreuse et inférieure à une température de fusion de la première matière première fibreuse utilisée, de manière à réticuler les fibres, **caractérisé en ce que** les fibres ou les structures de fibres sont équipées d'un catalyseur acide avant l'étuvage.

**2.** Procédé selon la revendication 1, **caractérisé en ce que** le catalyseur acide est un acide de Lewis et/ou un acide protonique, de préférence un acide organique, de manière encore davantage préférée un acide carboxylique en $C_{1-10}$, notamment un acide carboxylique en $C_2$-$C_6$.

**3.** Procédé selon la revendication 2, **caractérisé en ce que** l'acide protonique est choisi dans le groupe constitué par l'acide acétique, l'acide formique, l'acide propionique, l'acide citrique, l'acide benzoïque, l'acide para-toluènesulfonique et/ou l'acide de Lewis est choisi dans le groupe constitué par les ions de métaux bi- ou trivalents, notamment Zn(II) et Al (III).

**4.** Procédé selon une ou plusieurs des revendications 1 à 3, **caractérisé en ce que** l'équipement des fibres ou des structures de fibres en la première matière première fibreuse avec le catalyseur acide est réalisé au moyen d'une solution ou d'une suspension du catalyseur acide dans un solvant, notamment choisi parmi l'éthanol, le méthanol ou l'isopropanol.

**5.** Procédé selon la revendication 4, **caractérisé en ce que** la solution ou la suspension contenant le catalyseur acide est appliquée par foulardage, pulvérisation, plaquage et/ou imprégnation de mousse sur les fibres ou les structures de fibres.

**6.** Procédé selon une ou plusieurs des revendications 1 à 5, **caractérisé en ce qu'**une solution ou une suspension qui contient le catalyseur acide en une quantité de 0,01 à 10 % en poids, de préférence de 0,05 à 5 % en poids, notamment de 0,1 à 1 % en poids, à chaque fois par rapport au poids total du solvant, est utilisée.

**7.** Procédé selon une ou plusieurs des revendications 1 à 6, **caractérisé en ce que** la quantité de catalyseur acide appliquée sur les fibres ou la structure de fibres de 0,01 à 15 % en poids, de préférence de 0,05 à 10 % en poids, notamment de 0,1 à 1 % en poids, à chaque fois par rapport au poids des fibres.

**8.** Procédé selon une ou plusieurs des revendications 1 à 7, **caractérisé en ce que** le solvant est éliminé par séchage après l'application de la solution ou de la suspension contenant le catalyseur acide.

**9.** Procédé selon une ou plusieurs des revendications 1 à 8, **caractérisé en ce que** la durée d'étuvage prédéterminée est de 1 minute à 0,5 h, de préférence de 1 minute à 15 minutes, de manière encore davantage préférée de 1 minute à 10 minutes, de manière encore davantage préférée de 1 minute à 5 minutes et notamment de 1 minute à 3 minutes.

**10.** Procédé selon une ou plusieurs des revendications 1 à 9, **caractérisé en ce qu'**un procédé de solidification pour la fabrication d'une structure de fibres bi- ou tridimensionnelle, notamment pour la fabrication d'un non-tissé, est réalisé avant l'équipement avec le catalyseur acide.

**11.** Procédé selon une ou plusieurs des revendications 1 à 10, **caractérisé en ce que** d'autres fibres en au moins une deuxième matière première fibreuse sont incorporées.

12. Fibres ou structures de fibres hydrogélifiantes, mono-, bi- ou tridimensionnelles, fabriquées par un procédé selon une ou plusieurs des revendications 1 à 11, un acide non volatil dans les conditions de l'étuvage étant utilisé en tant que catalyseur acide.

13. Utilisation de fibres ou de structures de fibres selon la revendication 12 pour la fabrication de matériaux pour des applications médicales, de gazes pour plaies, de bandages pour plaies, de matériaux de suture, d'implants, d'échafaudages d'ingénierie tissulaire, de médicaments, de matériaux supports, de matériaux isolants, de matériaux de filtration, de produits absorbants techniques, de produits pour le domaine alimentaire, de produits d'hygiène, de produits cosmétiques, de produits ménagers, les produits d'hygiène comprenant les produits d'hygiène féminine, les couches et les produits pour l'incontinence, les produits ménagers comprenant les matériaux de nettoyage.

14. Bandages pour plaies ou gazes pour plaies contenant des fibres ou des structures de fibres selon la revendication 12.

Fig.1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0130407 A1
- WO 2005103097 A1
- JP H03279410 B
- WO 2012048768 A

**In der Beschreibung aufgeführte Nicht-Patentliteratur**

- **AHMET et al.** Properties of electrospun poly(vinyl alcohol) hydrogel nanofibers crosslinked with 1,2,3,4-butanetetracarboxylic acid. *JOURNAL OF APPLIED POLYMER SCIENCE,* 18. Februar 2013, vol. 129 (6), 3140-3149
- *J Mater Sci,* 2010, vol. 45, 2456-2465